(19) Europäisches Patentamt
European Patent Office
Office européen des brevets

(11) Veröffentlichungsnummer: **0 021 207**
**B1**

(12) # EUROPÄISCHE PATENTSCHRIFT

(45) Veröffentlichungstag der Patentschrift: **07.08.85**

(51) Int. Cl.$^4$: **C 07 D 207/337,**
A 61 K 31/40, C 07 D 261/18

(21) Anmeldenummer: **80103180.8**

(22) Anmeldetag: **09.06.80**

(54) **Alphacarbamoyl-pyrrolpropionitrile, Verfahren zu ihrer Herstellung, sowie pharmazeutische Präparate die diese Verbindungen enthalten.**

(30) Priorität: **11.06.79 US 46063**

(43) Veröffentlichungstag der Anmeldung:
**07.01.81 Patentblatt 81/01**

(45) Bekanntmachung des Hinweises auf die Patenterteilung:
**07.08.85 Patentblatt 85/32**

(84) Benannte Vertragsstaaten:
**AT BE CH DE FR GB IT LI LU NL SE**

(56) Entgegenhaltungen:
**BE-A- 842 688**
**BE-A- 842 689**
**BE-A- 861 500**
**US-A-3 642 794**

(73) Patentinhaber: **CIBA-GEIGY AG**
**Postfach**
**CH-4002 Basel (CH)**

(72) Erfinder: **Walker, Gordon Northrop, Dr.**
**Lake Trail West**
**Mount Kemble Lake Morristown New Jersey (US)**

(74) Vertreter: **Zumstein, Fritz, Dr. et al**
**Bräuhausstrasse 4**
**D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 021 207 B1

**Beschreibung**

In besonderer Weise substituierte "Cyanessigsäure-anilide" und verwandte "Isoxazolyl-carbonsäureanilide", z.B. solche der Formeln

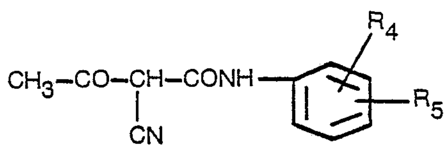 und 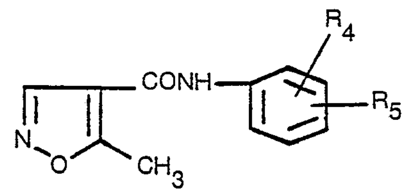

sind gemäss den belgischen Patenten 842,688; 842,689; 849,343; 861,500 oder dem US—Patent 4,061,767 "nicht-ulcerogene antiphlogistische und analgetische Mittel".

Es ist überraschend gefunden worden, dass die Pyrrolpropionitrile welche gegenstand dieses Patentes sind gegenüber den genannten bekannten Aniliden, z.B. in Hinsicht auf das Aktivitätsspektrum und die Verträglichkeit, ausgeprägte therapeutische Vorteile bieten. Für die aus J. Am. Chem. Soc., *35*, 959 (1913) bekannten analogen Benzoyl-Verbindungen sind keine pharmakologischen Wirkungen publiziert worden.

Die vorliegende Erfindung betrifft substituierte β-Oxo-α-phenylcarbamoyl-pyrrolpropionitrile der allgemeinen Formel I

$$\text{Pl—CO—}\overset{\text{CN}}{\overset{|}{\text{CH}}}\text{—CONH—Ph} \qquad \text{(I)},$$

worin Pl einen 2- oder 3-Pyrrolylrest bedeutet, der in 1-Stellung durch $C_1$—$C_7$-Alkyl oder Ph-$C_1$—$C_7$-Alkyl substituiert ist und in den übrigbleibenden drei Stellungen unsubstituiert oder durch eine bis drei, gleiche oder verschiedene $C_1$—$C_7$-Alkylgruppen und/oder eine Carboxy- oder Carbo-$C_1$—$C_7$-alkoxygruppe substituiert ist; und Ph Phenyl bedeutet, das unsubstituiert oder durch einen bis drei, gleiche oder verschiedene Substituenten der Gruppe $C_1$—$C_7$-Alkyl, $C_1$—$C_7$-Alkoxy, $C_1$—$C_7$-Alkylthio, Hydroxy, Halogen, Trifluormethyl, Nitro, Amino oder $C_1$—$C_7$-Alkanoylamino substituiert ist; diese Verbindungen in ihrer Enol-Form, ihre Enol-$C_1$—$C_7$-alkyläther oder Enol-$C_1$—$C_7$-alkanoylester, oder ihre Salze mit Basen, insbesondere ihre therapeutisch verwendbaren Salze mit Basen, Verfahren zu ihrer Herstellung, pharmazeutische Präparate die diese Verbindungen enthalten sowie diese Verbindungen zur therapeutische Verwendung.

Beide der genannten Pyrrolyl- und Phenylgruppe Pl und Ph sind vorzugsweise C-unsubstituiert. Sie können aber auch, insbesondere durch einen oder zwei, gleiche oder verschiedene Substituenten der Gruppe $C_1$—$C_7$-Alkyl, z.B. Methyl, Aethyl, n-Propyl oder i-Propyl oder -Butyl substituiert sein. Ueberdies kann der Rest Ph auch durch $C_1$—$C_7$-Alkoxy, z.B. Methoxy, Aethoxy, n- oder i-Propoxy oder -Butoxy; $C_1$—$C_7$-Alkylthio, z.B. Methylthio oder Aethylthio; Hydroxy; Halogen, z.B. Fluor, Chlor oder Brom; Trifluormethyl; Nitro; Amino oder $C_1$—$C_7$-Alkanoylamino, z.B. Acetylamino oder Propionylamino, substituiert sein. Der Rest Pl kann auch eine Carboxy oder Carbo-$C_1$—$C_7$-alkoxygruppe, z.B. Carbomethoxy oder Carbäthoxy, insbesondere in 3- oder 4-Stellung, enthalten.

In 1-Stellung des Pyrrolringes sind die $C_1$—$C_7$-Alkyl- oder Ph-$C_1$—$C_7$-Alkylgruppen vorzugsweise Methyl, aber auch Aethyl, n- oder i-Propyl oder -Butyl, Benzyl, 1- oder 2-Phenyläthyl, 1-, 2- oder 3-Phenylpropyl. Die letztgenannten Reste können unsubstituiert oder, wie für Ph angegeben, substituiert sein.

Von den $C_1$—$C_7$-Alkylgruppen sind diejenigen mit höchstens 4, insbesondere 1 oder 2 Kohlenstoffatomen bevorzugt.

Die β-Hydroxy tautomeren Verbindungen der Formel I sind genügend sauer, um die genannten Enol-$C_1$—$C_7$-alkyläther, Enol-$C_1$—$C_7$-alkanoylester, oder Salze mit Basen, insbesondere therapeutisch verwendbare Salze mit Basen, zu bilden. Diese Salze werden abgeleitet von einem Alkalimetall, Erdalkalimetall, Kupfer- oder Zinkhydroxyd; Ammoniak, von Mono-, Di- oder Tri-$C_1$—$C_7$-alkyl oder hydroxy-$C_1$—$C_7$-alkyl)-aminen, $C_1$—$C_7$-Alkylenaminen oder $C_1$—$C_7$-Alkylendiaminen. Solche Salze sind z.B. Natrium, Kalium-, Magnesium-, Ammonium-, Mono-, Di- oder Tri-(methyl, äthyl oder hydroxyäthyl)-ammonium-, Pyrrolidinium-, Aethylendiammonium- oder Morpholiniumsalze oder ihre verschiedenen Hydrate.

Die Verbindungen der Erfindung ziegen wertvolle pharmakologische Eigenschaften, in erster Linie antiinflammatorische und antiarthritische Wirkungen. Diese können in in-vitro oder in-vivo Versuchen nachgewiesen werden. In den letzteren werden vorzugsweise Säugetiere, z.B. Ratten, Katzen, Meerschweinchen oder Hunde als Testobjekte verwendet. Die Verbindungen der Erfindung können ihnen enteral, vorzugsweise oral, parenteral, z.B. subkutan oder intravenös, oder topisch, z.B. in Form von Lösungen in Wasser oder Oel, oder in Form von Stärke enthaltenden Suspensionen, verabreicht werden. Die verwendete Dosis kann in einem Bereich von zwischen 0,1 und 100 mg/kg/Tag, vorzugsweise 1 und 50 mg/kg/Tag, in erster Linie 2 und 25 mg/kg/Tag, liegen. Die für die genannten Wirkungen gewählten Tests sind entweder klassische Versuchsmethoden, z.B. der Carrageenin-Pfotenödem- oder der Adjuvansarthritis-Test in der Ratte, der Synoviitis-Versuch am Hund oder der Test, in welchem das durch ultraviolettes Licht hervorgerufene Erythem ausgewertet wird, oder moderne Testmethoden. Solche sind die neutrale Protease Hemmung [beschrieben in Arthritis Rheum. *17*, 47 (1974)] oder die Hemmung der Leukozyten Chemotaxis [N.Y. Acad. Sci., *256*, 177 (1975)]. Weitere Methoden sind die Abnahme der neutrophilen Adherenz [Amer. J. Med. *61*, 597 (1976)] oder die Hemmung der Prostaglangin-Synthetase, welcher Test in Biochem. *10*, 2372 (1971) beschrieben ist.

Illustrativ ist die folgende Testmethode, welche die sachdienlichen Angaben liefert: Männliche Charles River-Ratten von einem Gewicht von 250—300 g werden durch intradermale Injektionen von 0,1 ml Bacillus Calmette Guerin-Vakzine (BCG) immunisiert. Nach einer Woche wird den Versuchstieren, zwecks Auslösung der Anreicherung von Makrophagen, eine Injektion von 10 ml steriler 2%iger Reis-Stärke-Lösung intraperitoneal verabreicht. Am elften Tag nach der Immunisierung werden die Tiere getötet und peritoneale Makrophagen mit 20 ml Gey'scher gepufferter Salzlösung, welche Heparin enthält (25 Einheiten/ml), gesammelt. Die Zellen werden 10 Minuten bei 1000 Umdrehungen/Minute zentrifugiert, mit weiteren 50 ml Gey'scher Lösung bei gleicher Geschwindigkeit und Zeit gewaschen und dann in Gey'scher Lösung, welche 0,1% Human-Serumalbumin (Fraktion V, Sigma Co., pH = 7,1) enthält, um eine Konzentration von $2 \times 10^6$ Zellen/ml zu erreichen, suspendiert.

Mit den Test-Substanzen werden $1 \times 10^{-2}$ Mol-Lösungen in Dimethylacetamid hergestellt. Nachfolgende Verdünnungen werden mit Gey'scher Lösung gemacht und diese schliesslich zu der obigen Zellen-Suspension gegeben, um geeignete Endkonzentrationen von $10^{-4}$, $10^{-5}$, $10^{-6}$ und $10^{-7}$ Mol zu erreichen. Nach dem Einbringen der Suspensionen in den oberen Teil von modifizierten Boyden-Chemotaxis-Kammern, verbleiben die genannten Test-Substanzen mit den Zellen zusammen.

Als chemotaktisches Mittel wird ein Rattenserum, das mit dem Lipopolysaccharid von Escherichia coli aktiviert ist (Difco), (1/10 Verdünnung bei pH = 7,1), verwendet. Dieses Serum wird in die unteren Teile der genannten Kammern eingefüllt. Der die Zellen enthaltende Teil der Kammer ist von der chemotaktischen Lösung durch eine Zellulose-Filtermembran, welche Poren von 8 Mikron aufweist, getrennt. Die Kammern werden in dreifacher Wiederholung aufgestellt und 5 Stunden bei 37°C inkubiert. Als Kontrolle der Zellenwanderung werden Zellensuspensionen, welche keine Test-Verbindung enthalten, eingesetzt. Nach der Inkubation werden die Filter entfernt, fixiert und mit Eisenhämatoxylin nach Weigert gefärbt. Vier Felder der unteren Oberfläche des Filters werden bei einer 320-fachen Vergrösserung mikroskopisch untersucht. Als Index für die chemotaktische Aktivatät wird der Durchschnitt der Anzahl der in jenen vier Feldern gezählten Neutophilen verwendet. In diesem Testverfahren werden auch Indomethacin und Levamisol als Bezugsverbindungen eingesetzt.

So sind im Adjuvansarthritis-Test z.B. das 1-Methyl-β-oxo-α-phenylcarbamoyl-2-pyrrolpropionitril, ein typisches Mitglied der erfindungsgemässen Verbindungen oder die genannten Enoläther oder Enolester, Salze oder ihre verwandten Verbindungen der Formeln I und II hoch aktiv in Ratten bei peroralen Dosen bis hinunter zu 2 mg/kg/Tag. Der Einfluss dieser Verbindungen auf die in-vitro chemotaktische Aktivität von BCG-immunisierten Makrophagen ist bei Konzentrationen bis hinunter zu $10^{-5}$ und $10^{-6}$ Mol signifikant und er äussert sich in der Steigerung der chemotaktischen Reaktion der Makrophagen. Demgegenüber beeinflusst das Indomethacin die Makrophagen-Chemotaxis nicht, während das Levamisol, ein bekannter Immunopotentiator, eine erhöhte Wanderung bei Endkonzentrationen von $10^{-3}$ Mol bis $10^{-5}$ Mol hervorruft.

Die obigen Testmethoden zeigen, dass die erfindungsgemässen Verbindungen eine sehr gute antiinflammatorische Wirkung entfalten, aber auch einen im Vergleich zu den üblichen nicht-steroidalen Antiinflammatorika abweichenden Wirkungsmechanismus besitzen, indem sie auch eine dem Levamisol ähnliche Wirkungskomponente enthalten, wie sich aus dem Makrophagen-Chemotaxis-Test ergibt. [Levamisol ist ein krankheitsbeeinflussendes antirheumatisches Arzneimittel. (Es sei diesbezüglich auf die Zeitschriften "Arthritis and Rheumatism", *18*, 385 (1975) und "The Lancet", 21. Februar 1976, Seite 393, verwiesen)]. Die Verbindungen der vorliegenden Erfindung, welche sowohl die Aktivität vom Indomethacin-Typus als auch diejenige vom Levamisol-Typus aufweisen, sind wertvolle antiinflammatorische und antiarthritische Mittel, z.B. zur Behandlung oder Kontrolle von entzündlichen arthritischen und/oder dermatopathologischen Zuständen. Die neuen Verbindungen können überdies als Zwischenprodukte zur Herstellung von anderen wertvollen, insebesondere von pharmakologischen wirksamen Präparaten, eingesetzt werden.

Bevorzugte Verbindungen sind diejenigen der Formel I, worin Pl 1-($C_1$—$C_7$-Alkyl oder Ph-$C_1$—$C_7$-Alkyl)-2- oder -3-pyrrolyl bedeutet, welches in den übrigbleibenden Stellungen unsubstituiert oder durch eine oder zwei $C_1$—$C_7$-Alkylgruppen, oder eine Carboxy- oder Carbo-$C_1$—$C_7$-alkoxygruppe substituiert ist, Ph für Phenyl steht, das unsubstituiert oder durch einen oder zwei, gleiche oder verschiedene Substituenten der Gruppe $C_1$—$C_7$-Alkyl, $C_1$—$C_7$-Alkoxy, $C_1$—$C_7$-Alkylthio, Hydroxy, Halogen, Trifluormethyl, Nitro, Amino und $C_1$—$C_7$-Alkanoylamino substituiert ist, ihre Enol-$C_1$—$C_7$-alkyläther oder Enol-$C_1$—$C_7$-alkanoylester, oder ihre Salze mit Basen, insbesondere ihre therapeutisch verwendbaren Salze mit Basen.

Besonders hervorzuheben sind Verbindungen der allgemeinen Formel II

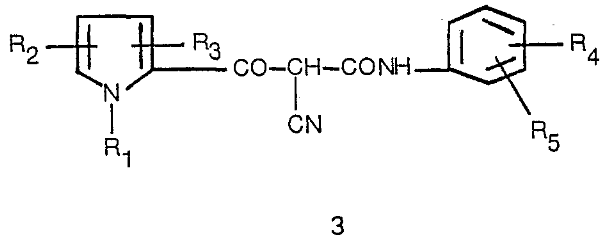

(II),

worin $R_1$ $C_1$—$C_7$-Alkyl bedeutet, jedes der Symbole $R_2$ und $R_3$ für Wasserstoff oder $C_1$—$C_7$-Alkyl steht, und jedes der Symbole $R_4$ und $R_5$ Wasserstoff, $C_1$—$C_7$-Alkyl, $C_1$—$C_7$-Alkoxy, Hydroxy, Halogen oder Trifluormethyl bedeutet, oder ihre Salze mit Basen, insbesondere ihre therapeutisch verwendbaren Salze mit Basen.

Bevorzugt sind weiter Verbindungen der Formel II, worin $R_1$ Alkyl mit höchstens 4 Kohlenstoffatomen bedeutet, jedes der Symbole $R_2$ und $R_3$ für Wasserstoff steht, und jedes der Symbole $R_4$ und $R_5$ Wasserstoff, Alkyl oder Alkoxy, jeweils mit höchstens 4 Kohlenstoffatomen, Fluor, Chlor oder Trifluormethyl bedeutet, oder ihre Salze mit einer Base, insbesondere ihre therapeutisch verwendbaren Salze mit Basen.

Besonders bevorzugt sind Verbindungen der allgemeinen Formel II, worin $R_1$ Methyl bedeutet, jedes der Symbole $R_2$ und $R_3$ für Wasserstoff steht, und jedes der Symbole $R_4$ und $R_5$ Wasserstoff, Methyl, Methoxy, Fluor, Chlor oder Trifluormethyl bedeutet, oder ihre Natrium-, Kalium-, Calcium-, Triäthylammonium- oder Trihydroxyäthylammoniumsalze, wobei eines der Symbole $R_4$ und $R_5$, welches von Wasserstoff verschieden ist, vorzugsweise in der para-Stellung steht.

Die Verbindungen der Erfindung werden nach an sich bekannten Methoden, z.B. dadurch hergestellt, dass man

a) Verbindung der Formeln

$$Pl—CO—CH_2—CN \text{ und } OC=N—Ph$$

addiert, oder

b) Verbindungen der Formeln

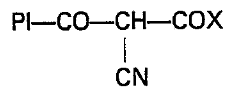

und H—NH—Ph,

worin X $C_1$—$C_7$-Alkoxy, $C_1$—$C_7$-Alkanoyloxy oder Halogen bedeutet, kondensiert oder

c) Verbindungen der Formeln

Pl—Y und 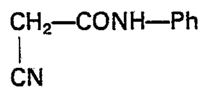

worin Y $C_1$—$C_7$-Alkoxycarbonyl, Halogencarbonyl oder Cyan bedeutet, kondensiert und die erhaltenen Imine hydrolysiert oder

d) Verbindung der Formel

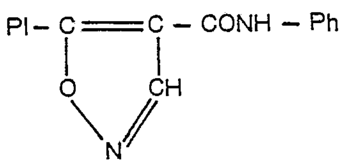

mit einer starken Base isomerisiert, und, wenn erwünscht, eine erhaltene Verbindung der Formel I in eine andere Verbindung der Erfindung umwandelt, und/oder, wenn erwünscht, ein erhaltenes Enol in ein Enol-$C_1$—$C_7$-alkyläther, oder Enol-$C_1$—$C_7$-alkanoylester überführt, und/oder, wenn erwünscht, ein erhaltenes Enol in ein Salz mit einer Base oder ein erhaltenes Enolsalz in das freie Enol oder in ein anderes Salz mit einer Base überführt, und/oder, wenn erwünscht, ein erhaltenes Gemisch von Isomeren in die einzelnen Isomeren auftrennt.

Die Addition des Isocyanats im Verfahren a) wird vorzugsweise gemäss den genannten belgischen Patenten oder gemäss dem US-Patent 3,905,997, d.h. in Abwesenheit oder in Gegenwart einer anorganischen oder organischen Base, z.B. Natiumhydrid, oder in Gegenwart oder in Abwesenheit eines polaren Lösungsmittels, z.B. eines Aethers wie Diäthyläther oder Tetrahydrofuran, und/oder eines Amids oder Sulfoxids, z.B. Dimethylformamid oder Dimethylsulfoxid, vorzugsweise bei erhöhten Temperaturen, z.B. bei ungefähr 150°, wenn keine Base verwendet wird, durchgeführt.

Das erfindungsgemässe Verfahren wird vorzugsweise wie folgt vorgenommen: Die Suspension des genannten Nitrils in einem aromatischen Kohlenwasserstoff, z.B. in warmem Toluol, wird mit einem geringen molaren Ueberschuss eines wasserfreien Tri-niederalkylamins, vorzugsweise Triäthylamins behandelt. Dann wird dazu ein Moläquivalent des Phenylisocyanats Ph—N=CO oder seine Lösung in einem vorher genannten polaren Lösungsmittel, z.B. Dimethylsulfoxid, gegeben. Das Reaktionsgemisch wird 2—12 Stunden bei Zimmertemperatur gerührt und sein Volumen unter Erwärmen bei nicht zu hohen Temperaturen, z.B. bis 100°, vermindert. Der erhaltene Niederschlag wird in einem Alkanol, z.B. Methanol aufgenommen und die Lösung mit überschüssiger, verdünnter, wässeriger Säure, z.B. mit 0,3-normaler Salzsäure, behandelt. Das erhaltene Rohprodukt wird abgetrennt, mit Wasser gewaschen, getrocknet, trituriert und/oder aus geeigneten Lösungsmitteln umkristallisiert. Solche Lösungsmittel sind Niederalkanole, Niederalkanone, Di-niederalkyläther und/oder Niederalkyl-niederalkanoate, z.B. Methanol, Aceton, Diäthyläther und/oder Essigsäureäthylester.

Die Aminierung nach dem Verfahren b) wird auch in üblicher Weise, vorzugsweise zwischen Zimmertemperatur und ungefähr 150° durchgeführt. Man arbeitet entweder mit äquivalenten Mengen der Reaktionspartner, vorzugsweise wenn ein Ester verwendet wird, oder mit einem Aminüberschuss, oder in

Gegenwart einer anderen Base. Solche Basen sind z.B. tertiäre Amine, wie ein Tri-niederalkylamin oder Pyridin. Diese werden bei der Verwendung von Halogenid- oder Anhydrid-Ausgangsstoffen für die Neutralisation der gebildeten Säure, eingesetzt. Das in der Reaktion von Ester-Ausgangsstoffen entstandene Niederalkanol wird vorzugsweise zusammen mit dem Lösungsmittel, z.B. mit einem aromatischen Kohlenwasserstoff, z.B. Benzol, Toluol oder Xylol, abdestilliert.

Die Kondensation im Verfahren c) wird vorzugsweise unter Verwendung von Alkalimetallen, ihren Niederalkoxiden oder insbesondere Hydriden, z.B. Natriumhydrid, in den genannten polaren Lösungsmitteln, vorzugsweise Dimethylformamid oder Dimethylsulfoxid, vorgenommen. Diese Kondensation ist analog zur Herstellung der genannten Nitrilausgangsstoffe für die Verfahren a) und b), welche Kondensation in den entsprechenden Beispielen illustriert ist.

Schliesslich wird die Isomerisierung im Verfahren d) in Gegenwart von starken anorganischen oder organischen Basen, z.B. Alkalimetallhydroxyden oder Tri-niederalkyl-aralkylammonium-hydroxyden, z.B. Trimethyl-benzyl-ammoniumhydroxyd, durchgeführt.

Die erhaltenen Verbindungen der Formel I können in an sich bekannter Weise ineinander übergeführt werden. So können z.B. erhaltene Enole, vorzugsweise mit Diazoalkanen, veräthert, oder z.B. mit $C_1$—$C_7$-Alkansäureanhydriden, verestert werden. Therapeutisch verwendbare Salze der genannten Enole können z.B. mit wässerigen Alkalimetallhydroxyden, vorzugsweise in Gegenwart eines Aethers oder Alkohols als Lösungsmittel, z.B. eines Niederalkanols, hergestellt werden. Aus den alkoholischen Lösungen können die Salze mit den genannten Aethern, z.B. Diäthyläther oder Tetrahydrofuran, ausgefällt werden. Man erbeitet bei mässigen Temperaturen, z.B. unter 100°. Erhaltene Salze können, wie oben erwähnt, durch Behandlung mit Säuren in die freien Verbindungen umgewandelt werden. Auch eine Nitrogruppe im Rest Ph kann z.B. mit katalytisch aktiviertem Wasserstoff, wie Wasserstoff in Gegenwart von Nickel- oder Palladium-Katalysatoren, in die Aminogruppe übergeführt werden. Letztere kann entweder wie oben beschrieben oder mit $C_1$—$C_7$-Alkansäurehalogeniden oder $C_1$—$C_7$-Alkylestern, acyliert werden.

Die Ausgangsstoffe sind bekannt, oder, sie können soweit neu gemäss den für analoge Verbindungen beschriebenen Verfahren, z.B. denjenigen die im Stand der Technik für die dort genannten Verbindungen beschrieben sind, oder wie in den Beispielen illustriert, hergestellt werden.

Ausgangsstoffe und Endprodukte, welche Isomerengemische sind, können nach an sich bekannten Methoden, z.B. durch fraktionierte Destilation, Kristallisation und/oder Chromatographie, in die einzelnen Isomeren getrennt werden.

Die oben genannten Reaktionen werden nach an sich bekannten Methoden, in Gegenwart oder Abwesenheit von Verdünnugsmitteln, vorzugsweise in solchen, welche gegenüber den Reagenzien inert sind und diese lösen, Katalysatoren, Kondensationsmitteln oder Neutralisationsmitteln, und/oder in einer inerten Atmosphäre, unter Kühlung, bei Zimmertemperatur oder bei erhöhten Temperaturen, bei normalem oder erhöhtem Druck durchgeführt.

Die Erfindung betrifft ebenfalls Abänderungen des vorliegenden Verfahrens, wonach ein auf irgendeiner Stufe des Verfahrens erhältliches Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder eines reaktionsfähigen Derivates, vorzugweise eines Alkalimetall- oder Trialkylammoniumsalzes, verwendet wird. Die genannten Isocyanate können auch ausgehend von entsprechenden Säureaziden, und gemischte Anhydride aus entsprechenden Säuren und einfachen Alkansäureanhydriden, hergestellt werden.

Beim Verfahren der vorliegenden Erfindung werden vorzugsweise solche Ausgangsstoffe verwendet, welche zu den eingangs als besonders wertvoll geschilderten Verbindungen, insbesondere solchen der Formel II führen.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z.B. Laktose, Dextrose, Rohrzucker, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder das Natriumsalz davon, Enzyme der Bindemittel und/oder Brausemischungen, oder Adsorptionsmittel, Farbstoffe, Geschmackstoffe und Süssmittel. Injizierbare Präparate sind vorzugsweise isotonische wässerige Lösungen oder Suspensionen, und Suppositorien oder topische Lotionen in erster Linie Fettemulsionen oder -suspensionen. Die pharmakologischen Präparate können sterilisiert sein und/oder Hilfsstoffe, z.B. Konservier-, Stabiliser-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren, hergestellt und enthalten von 1% bis 75%, insbesondere von 1% bis 50%, des Aktivstoffes.

Die folgenden Beispiele dienen zur Illustration der Erfindung. Temperaturen werden in Celsiusgraden

angegeben und die Angaben über Teile betreffen Gewichtsteile. Wenn nicht anders definiert, wird das Eindampfen von Lösungsmitteln unter atmosphärischem Druck durchgeführt.

Beispiel 1

Eine Suspension von 12,9 g 1-Methyl-β-oxo-2-pyrrolpropionitril in 150 ml trockenem Toluol und 10,1 g wasserfreiem Triäthylamin wird unter Rühren mit 10,7 g Phenylisocyanat versetzt. Nach der Auflösung von festen Stoffen wird die dunkelrote Lösung 30 Minuten bei Zimmertemperatur, 5 Minuten auf dem Dampfbad und über Nacht bei Zimmertemperatur gehalten. Das Gemisch wird auf dem Dampfbad eingedampft, der Rückstand in Methanol aufgenommen und die Lösung in das Gemisch von 25 ml 5-normaler Chlorwasserstoffsäure und 600 ml Wasser gegossen. Die erhaltenen hellbraunen Kristalle werden abgetrennt, mit Wasser gewaschen, mit Aethanol trituriert und aus ungefähr 2200 ml Methanol umkristallisiert. Man erhält das 1-Methyl-β-oxo-α-phenylcarbamoyl-2-pyrrol-propionitril der Formel

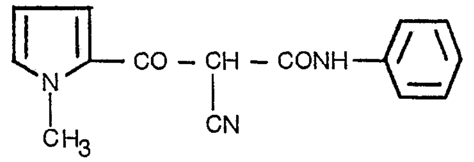 ,

welches bei 174—175° schmilzt.

Der Ausgangsstoff wird wie folgt hergestellt: Eine Lösung von 28 g 1-Methylpyrrol-2-carbonsäure in 20 ml Dimethylformamid wird zu einer Suspension, welche ausgehend von 12 g 50%igem Natriumhydrid in Mineralöl durch Waschen mit Petroläther und Suspendieren in 50 ml Dimethylformamid hergestellt ist, unter Rühren und Kühlen gegeben. Das Reaktionsgemisch wird dann mit 30 ml Methyljodid versetzt und bei Zimmertemperatur weitergerührt. Nach dem Abklingen der anfänglich exothermischen Reaktion gibt man weitere 10 ml Methyljodid dazu. Man lässt das Reaktionsgemisch über Nacht stehen, behandelt es mit Wasser und extrahiert es mit Diäthyläther. Der Extrakt wird mit wässeriger Natriumcarbonatlösung und Wasser gewaschen, getrocknet und eingedampft. Man erhält den öligen 1-Methylpyrrol-2-carbonsäure-methyl-ester.

Eine Lösung von 28 g der letztgenannten Verbindung in 50 ml Acetonitril wird unter Rühren zu einer Suspension, welche aus 19 g 50%igem Natriumhydrid in Mineralöl, durch Waschen mit Petroläther und Suspendieren in 50 ml Dimethylformamid hergestellt ist, gegeben. Nach dem Abklingen der exothermischen Reaktion wird das Gemisch 15 Minuten auf dem Dampfbad erhitzt, wobei eine weitere lebhaft sprudelnde Reaktion stattfindet. Die dickflüssige, rotbraune Suspension wird mit weiteren 50 ml Dimethylformamid verdünnt und über Nacht bei Zimmertemperatur stehen gelassen. Das Gemisch wird dann auf dem Dampfbad 5 Minuten wieder erwärmt, dann gekühlt und mit Wasser verdünnt. Die wässerige Lösung wird filtriert, einmal mit Diäthyläther gewaschen und mit Chlorwasserstoffsäure angesäuert. Die erhaltenen Kristalle werden abgetrennt, mit Wasser gewaschen, getrocknet, mit kaltem Aethanol trituriert und aus Aethanol umkristallisiert. Man erhält das 1-Methyl-β-oxo-2-pyrrolpropionitril, welches bei 107—109° schmilzt.

Beispiel 2

Durch Behandlung von äquivalenten Mengen konzentrierter wässerigen Lösungen von Natriumhydroxyd, Kaliumhydroxyd oder Calciumhydroxyd mit 1-Methyl-β-oxo-α-phenylcarbamoyl-2-pyrrolpropionitril, Filtrieren des Gemisches und Eindampfen des Filtrats zur Trockene, erhält man die drei entsprechenden Metallsalze. Diese scheinen keine gut definierten kristallinen Eigenschaften oder Schmelzpunkte zu haben, obwohl sie aus äthanolischer Lösung durch Zugabe von Diäthyläther wieder ausgefällt werden können. Die Salze sind wasserlöslich und sie ziegen im Infrarotspektrum die folgenden Banden:

Na-Salz: 4,57; 6,17 und 6,29 μ;
K-Salz: 4,58; 6,17 und 6,30 μ;
Ca-Salz: 4,55; 6,13 und 6,23 μ.

In jedem Fall wird durch Ansäuern der wässrigen Salzlösung mit Chlorwasserstoffsäure die identische freie Verbindung (F. 174—175°) zurückgewonnen.

Beispiel 3

Eine Suspension von 21,4 g 1-Methyl-β-oxo-α-phenylcarbamoyl-2-pyrrolpropionitril in 100 ml absolutem Aethanol wird mit 11,68 ml Trisäthanolamin in 25 ml Aethanol versetzt und bis zum Abschluss der Auflösung erwärmt. Die Lösung wird heiss filtriert, unter Rühren auf Zimmertemperatur abkühlen gelassen, die erhaltene Suspension auf 10° gekühlt, filtriert, und der Rückstand mit 25 ml kaltem Aethanol gewaschen. Man erhält das entsprechende Trishydroxyäthyl-ammoniumsalz, welches bei 115—117° schmilzt.

Beispiel 4

Man gibt 3,8 g 1-Methyl-β-oxo-α-phenylcarbamoyl-2-pyrrolpropionitril zu 500 ml ätherischem Diazomethan, welches aus 10,3 g N-Nitroso-N-methylharnstoff mit 35 ml 45%iger wässeriger Kaliumhydroxydlösung hergestellt und über solchen Tabletten getrocknet wird. Nach dem Abklingen der Stickstoffentwicklung wird die Lösung filtriert und eingedampft. Der Rückstand wird mit Diäthyläther trituriert und aus Essigsäureäthylester umkristallisiert. Man erhält den entsprechenden Methyl-enoläther, nämlich das 1-Methyl-β-methoxy-α-phenylcarbamoyl-2-pyrrolacrylnitril, welches bei 121—122° schmilzt.

Beispiel 5

Eine Suspension von 4 g 1-Methyl-β-oxo-2-pyrrolpropionitril in 70 ml Toluol und 3,2 g Triäthylamin wird unter Rühren mit 3,7 g p-Fluorphenylisocyanat versetzt. Das Gemisch wird auf dem Dampfbad bis zur Auflösung des festen Materials erwärmt und die rotbraune Lösung über Nachtbei Zimmertemperatur stehen gelassen. Das Reaktionsgemisch wird auf dem Dampfbad eingedampft, der Rückstand in Methanol aufgenommen und die Lösung in ein Gemisch von 8 ml 5-normaler Chlorwasserstoffsäure und 300 ml Wasser gegossen. Die ausgefallenen Kristalle werden abgetrennt, mit Wasser gewaschen, getrocknet, mit Methanol trituriert und aus Essigssäureäthylester umkristallisiert. Man erhält das 1-Methyl-β-oxo-α-(p-fluorphenylcarbamoyl)-2-pyrrolpropionitril, welches bei 198—199° schmilzt.

Beispiel 6

Ein Gemisch von 1,1 g 1-Methyl-β-oxo-α-äthoxycarbonyl-2-pyrrolpropionitril, 1,1 g Anilin und 60 ml Xylol wird 4,5 Stunden unter Rückfluss gekocht. Das Reaktionsgemisch lässt man über Nacht abkühlen und bei Zimmertemperatur stehen. Dann wird die Lösung filtriert, eingedampft und der Rückstand aus Methanol umkristallisiert. Man erhält das 1-Methyl-β-oxo-α-phenylcarbamoyl-2-pyrrolpropionitril, welches bei 173—174° schmilzt. Das Produkt ist mit demjenigen des Beispiels 1 identisch.

Der Ausgangsstoff wird wie folgt hergestellt: Eine Lösung von 10 g 1-Methylpyrrol-2-carbonsäure in 650 ml trockenem Diäthyläther und 8,5 g wasserfreiem Triäthylamin wird unter Rühren mit einer Lösung von 6 ml Thionylchlorid in 50 ml Diäthyläther stufenweise versetzt. Das Reaktionsgemisch wird nach 30 Minuten filtriert und der Rückstand mit Diäthyläther gewaschen. Das Filtrat wird auf ein kleineres Volumen eingedampft, wieder filtriert und schliesslich unter vermindertem Druck eingedampft. Man erhält das entsprechende Säurechlorid.

Seine Lösung in 20 ml Aethylenglykol-dimethyläther wird zu einer Suspension von 7,3 g 50%igem Natriumhydrid in Mineralöl (gewaschen mit Petroläther) in 50 ml Dimethylformamid und 20 g Cyanessigsäureäthylester unter Rühren und Kühlen langsam gegeben. Die mässig exothermische Reaktion wird durch kurzes Erwärmen des Gemisches auf dem Dampfbad beschleunigt. Das Reaktionsgemisch wird über Nacht stehen gelassen, mit Wasser behandelt, mit 5-normaler Chlorwasserstoffsäure angesäuert und mit Diäthyläther extrahiert. Der Extrakt wird mit Wasser gewaschen, getrocknet, eingedampft und der Rückstand trituriert und aus Diäthyläther umkristallisiert. Man erhält das 1-Methyl-β-oxo-α-äthoxycarbonyl-2-pyrrolpropionitril, welches bei 74—77° schmilzt.

Das gleiche Produkt wird durch Acylierung mit dem gemischten Anhydrid, d.h. durch Verwendung der äquivalenten Menge von Chlorameisensäureäthylester anstelle von Thionylchlorid, in der obigen Reaktionsstufe erhalten.

Beispiel 7

Ein Gemisch von 5,7 g 1-Methyl-β-oxo-α-äthoxycarbonyl-2-pyrrolpropionitril, 3,6 g p-Aminophenol und 300 ml Xylol wird 2 Stunden unter Rückfluss gekocht und heiss filtriert. Das Filtrat wird gekühlt, die erhaltenen Kristalle abgetrennt und aus Methanol umkristallisiert. Man erhalt das 1-Methyl-β-oxo-α-(p-hydroxyphenylcarbamoyl)-2-pyrrolpropionitril, welches bei 182—184° schmilzt.

Beispiel 8

Eine Suspension von 0,8 g 50%igem Natriumhydrid in Mineralöl (mit Petroläther gewaschen) in 10 ml 1,2-Dimethoxyäthan wird unter Rühren zuerst mit 2,9 g 1-Benzyl-β-oxo-2-pyrrolpropionitril in 30 ml 1,2-Dimethoxyäthan und dann mit 2,7 g p-Chlorphenylisocyanat versetzt. Das Reaktionsgemisch wird über Nacht bei Zimmertemperatur stehen gelassen, eingedampft, der Rückstand in Wasser aufgenommen, die Lösung filtriert und mit 5-normaler Chlorwasserstoffsäure angesäuert. Der erhaltene gelbe Niederschlag wird abgetrennt, mit Wasser gewaschen, mit Methanol trituriert und aus Essigsäureäthylester umkristallisiert. Man erhält das 1-Benzyl-β-oxo-α-(p-chlorphenylcarbamoyl)-2-pyrrolpropionitril, welches bei 214—215° schmilzt.

Der Ausgangsstoff wird wie folgt hergestellt: Man gibt 7,5 g Pyrrol-2-carbonsäuremethylester in 25 ml Dimethylformamid zu einer gerührten Suspension, welche aus 3,4 g 50%igem Natriumhydrid in Mineralöl, durch Waschen mit Petroläther und Suspendieren in 20 ml Dimethylformamid hergestellt ist. Das Gemisch wird dann mit 8,5 ml Benzylchlorid versetzt und über Nacht bei Zimmertemperatur gerührt. Das Reaktionsgemisch wird mit Wasser behandelt, mit Diäthyläther extrahiert, der Extrakt mit wässeriger Natriumhydroxydlösung und Wasser gewaschen, getrocknet und eingedampft. Man erhält den 1-Benzylpyrrol-2-carbonsäuremethylester als ein Oel. (Eine gereinigte Probe davon schmilzt bei 31—32°).

Eine Lösung von 9,3 g der letztgennanten Verbindung in 15 ml Acetonitril und 25 ml Dimethylformamid wird zu einer Suspension unter Rühren gegeben, welche aus 4 g 50%igem Natriumhydrid in Mineralöl, durch Waschen mit Petroläther und Suspendieren in 25 ml Dimethylformamid hergestellt wird. Das Gemisch wird 30 Minuten auf dem Dampfbad erwärmt, wobei sich eine dunkelrote Lösung bildet. Diese

wird über Nacht bei Zimmertemperatur stehen gelassen, mit Wasser versetzt, filtriert, mit Diäthyläther gewaschen und mit 5-normaler Chlorwasserstoffsäure angesäuert. Die erhaltenen Kristalle werden abgetrennt, mit Wasser gewaschen, getrocknet, mit Diäthyläther trituriert und aus Methanol umkristallisiert. Man erhält das 1-Benzyl-β-oxo-2-pyrrolpropionitril, welches bei 119—120° schmilzt.

Beispiel 9

Eine Lösung von 4 g Phenylcarbamoyl-acetonitril in 50 ml Dimethylformamid wird unter Rühren in einer Stickstoffatmosphäre mit 28 g Kalium-tert.-butoxid versetzt. Nach 2 Stunden wird die erhaltene Suspension auf 5° gekühlt und innerhalb 10 Minuten mit 4 g 1-Methylpyrrol-2-carbonsäurechlorid (US—A—3,551,571) versetzt. Das Reaktionsgemisch wird 18 Stunden bei Zimmertemperatur gerührt, in 300 ml Eiswasser gegossen und der erhaltene Niederschlag abfiltriert. Dieser wird mit Wasser gewaschen, getrocknet und aus Aethanol umkristallisiert. Man erhält das 1-Methyl-β-oxo-α-phenylcarbamoyl-2-pyrrolpropionitril, welches bei 172—174° schmilzt. Das Produkt ist mit demjenigen des Beispiels 1 identisch.

Der Ausgangsstoff wird wie folgt hergestellt: Ein Gemisch von 42,53 g Cyanessigsäure, 46,56 g Anilin und 500 ml Acetonitril wird unter Rühren in einer Stickstoffatmosphäre mit einer Lösung von 113,7 g N,N-Dicyclohexyl-carbodiimid in 500 ml Acetonitril innerhalb 30 Minuten versetzt. Nach 3 Stunden wird die erhaltene Suspension filtriert, der Rückstand mit 200 ml Acetonitril gewaschen und die Filtrate werden eingedampft. Der Rückstand wird mit 500 ml Diäthyläther und 50 ml Essigsäureäthylester trituriert, filtriert und getrocknet. Man erhält das Phenylcarbamoyl-acetonitril, welches bei 200—202° schmilzt.

Beispiel 10

Eine Suspension von 1,135 g 1-Methyl-β-oxo-2-pyrrolpropionitril in 17000 ml trockenem Toluol wird unter Rühren in einer Stickstoffatmosphäre zuerst mit 913 g wasserfreiem Triäthylamin und dann mit 913 g Phenylisocyanat versetzt. Die erhaltene dunkelbraune Lösung wird über Nacht bei Zimmertemperatur gerührt und dann bei 60—70°/10 mmHg eingedampft. Der Rückstand wird in 1400 ml Methanol aufgenommen und die Lösung mit 1400 ml 6-normaler Chlorwasserstoffsäure in 4200 ml Wasser behandelt. Die Suspension wird 20 Minuten auf 15—20° gekühlt, filtriert und der Rückstand zweimal mit 1800 ml Wasser, zweimal mit 1000 ml Isopropanol und 13 mal mit 1000 ml Diäthyläther gewaschen. Der rohe Rückstand wird bei 60° und 5 mmHg bis zur Gewichtskonstanz getrocknet. Man löst 1960 g dieses Rückstands in 44 400 ml Methylenchlorid bei Zimmertemperatur auf. Die Lösung wird mit 400 g Aktivkohle behandelt, filtriert und das Filtrat eingedampft. Der Rückstand wird mit 12000 ml wasserfreiem Aethanol trituriert, die Suspension bei 20° filtriert, viermal mit 1000 ml wasserfreiem Aethanol gewaschen und bei 60° und 5 mmHg getrocknet. Man erhält das 1-Methyl-β-oxo-α-phenylcarbamoyl-2-pyrrolpropionitril, welches bei 172—174° schmilzt. Das Produkt ist mit denjenigen der Beispiele 1, 6 oder 9 identisch.

Man suspendiert 1801 g der letztgenannten Verbindung in 10500 ml wasserfreiem Aethanol und gibt 1108 g Triäthanolamin dazu. Das Gemisch wird bis zur Auflösung des festen Materials gerührt und erhitzt und dann langsam auf 20° gekühlt. Der erhaltene Niederschlag wird abfiltriert, zweimal mit 1000 ml kaltem wasserfreiem Aethanol gewaschen und bei 45° und 0,1 mmHg getrocknet. Man erhält das entsprechende Tris-hydroxyäthylammonium-Salz, welches bei 115—117° schmilzt. Das Salz ist mit demjenigen des Beispiels 3 identisch.

Der Ausgangsstoff wird wie folgt hergestellt: Eine Lösung von 1325 g 1-Methylpyrrol-2-carbonsäure in 2400 ml Dimethylformamid wird unter Rühren in einer Stickstoffatmosphäre und Kühlung mit Eis zu einer Suspension gegeben, welche aus 568,5 g 50%igem Natriumhydrid in Mineralöl und 2400 ml Dimethylformamid hergestellt ist. Das Reaktionsgemisch wird dann zuerst mit 1000 ml Dimethylformamid und nachher mit 4316 g Methyljodid unter Rühren und bei einer Temperatur unter 88° versetzt. Das Rühren wird über Nacht bei Zimmertemperatur fortgesetzt, dann das Gemisch auf 10° gekühlt und mit 10600 ml Wasser versetzt. Das Gemisch wird dreimal mit 5300 ml Diäthyläther extrahiert, der Extrakt mit 5300 ml 10%igem wässerigem Natriumcarbonatlösung und 5300 ml Wasser gewaschen, getrocknet und eingedampft. Man erhält den öligen 1-Methylpyrrol-2-carbonsäuremethylester.

Eine Lösung von 1604 g der letztgenannten Verbindung in 2650 ml Acetonitril wird schnell zu einer Suspension gegeben, welche aus 1017 g 50%igem Natriumhydrid in Mineralöl und 2650 ml Dimethylformamid unter Rühren in einer Stickstoffatmosphäre hergestellt ist. Das Gemisch wird 2 Stunden auf 81° erhitzt und dann auf 10° gekühlt, schliesslich mit 25400 ml Wasser innerhalb 15 Minuten versetzt. Das Gemisch wird dreimal mit 9000 ml Diäthyläther gewaschen, 45 Minuten bei 10 mmHg zwecks Beseitigung des Diäthyläthers gerührt und mit 6400 ml 6-normaler Chlorwasserstoffsäure angesäuert. Die erhaltenen Kristalle werden abgetrennt, viermal mit 2400 ml Wasser und einmal mit 2400 Isopropanol gewaschen und bei 60° und 5 mmHg getrocknet. Man erhält das 1-Methyl-β-oxo-2-pyrrolpropionitril, welches bei 106—108° schmilzt.

Beispiel 11

Eine filtrierte Lösung von 4,4 g 2,6-Dichlorphenylisocyanat in 30 ml Toluol wird mit 2,5 g 1-Methyl-β-oxo-2-pyrrolpropionitril und 2,1 g Triäthylamin unter Rühren versetzt. Das Gemisch wird über Nacht bei Zimmertemperatur stehen gelassen und dann filtriert. Das feste Material wird mit Toluol und Diäthyläther gewaschen, in 50 ml Methanol aufgenommen und die Lösung in ein Gemisch von 6 ml 5-normaler Chlorwasserstoffsäure und 300 ml Wasser gegossen. Die erhaltenen Kristalle werden abgetrennt, mit Wasser

gewaschen, getrocknet, mit Aethanol trituriert und aus Methanol umkristallisiert. Man erhält das 1-Methyl-β-oxo-α-(2,6-dichlorphenylcarbamoyl)-2-pyrrolpropionitril, welches bei 196—199° schmilzt.

Beispiel 12

Eine Suspension von 2 g 1-Methyl-β-oxo-2-pyrrolpropionitril in 50 ml Toluol und 1,6 g Triäthylamin wird mit 2,1 g p-Methoxyphenylisocyanat behandelt. Das Gemisch wird bis zur Auflösung des festen Materials leicht erwärmt und über Nacht bei Zimmertemperatur stehen gelassen. Dann wird es eingedampft, der Rückstand in Methanol aufgenommen und mit 10 ml 10%iger wässeriger Natriumhydroxylösung und 250 ml Wasser versetzt. Die alkalische Lösung wird filtriert, mit 5-normaler Chlorwasserstoffsäure angesäuert und der erhaltene Niederschlag abgetrennt. Der Niederschlag wird mit Wasser gewaschen, getrocknet, mit warmem Gemisch von Methanol-Essigsäureäthylester trituriert und aus Essigsäureäthylester umkristallisiert. Man erhält das 1-Methyl-β-oxo-α-(p-methoxyphenylcarbamoyl)-2-pyrrolpropionitril, welches bei 192—193° schmilzt.

In analoger Weise wird auch das 1-Methyl-β-oxo-α-(p-methylthio-phenylcarbamoyl)-2-pyrrolpropionitril hergestellt. F.167—168°.

Beispiel 13

Eine Lösung von 4,9 g 1-Methyl-β-oxo-2-pyrrolpropionitril in 50 ml Toluol, 5 ml Dimethylsulfoxid und 4 g Triäthylamin wird mit einer Lösung von 5,7 g p-Nitrophenylisocyanat in einer minimalen Menge Toluol unter Rühren behandelt. Das Reaktionsgemisch wird über Nacht stehen gelassen, eingedampft und der Rückstand in Methanol aufgenommen. Die Lösung wird filtriert, mit Wasser und 6 ml Triäthylamin verdünnt, mit Essigsäureäthylester gewaschen, und die Waschflüssigkeit einmal mit 5%iger wässeriger Natriumhydroxydlösung extrahiert. Die vereinigten wässerigen Lösungen werden mit 5-normaler Chlorwasserstoffsäure angesäuert und der erhaltene Niederschlag abgetrennt. Dieser wird mit Wasser gewaschen, mit einem Gemisch von Methanol-Aethanol trituriert und aus Dimethylformamid umkristallisiert. Man erhält das 1-Methyl-β-oxo-α-(p-nitro-phenylcarbamoyl)-2-pyrrolpropionitril, welches bei 245—250° unter Zersetzung schmilzt.

Eine Lösung von 2 g der letztgenannten Verbindung in 220 ml Aethanol und 2 ml Triäthylamin wird über 0,5 g 10%iger Palladiumkohle bei 3,5 Atmosphären und Zimmertemperatur 50 Minuten hydriert. Die filtrierte Lösung wird eingdampft, der Rückstand in Wasser aufgenommen, mit 1 ml Triäthylamin versetzt und die Lösung mit Essigsäureäthylester und Diäthyläther gewaschen. Es wird mit 5-normaler Chlorwasserstoffsäure angesäuert, der Niederschlag abgetrennt, mit Wasser gewaschen, getrocknet und aus wässerigem Methanol umkristallisiert. Man erhält das 1-Methyl-β-oxo-α-(p-amino-phenylcarbamoyl)-2-pyrrolpropionitril, welches unter Zersetzung bei 193—195° schmilzt.

Man löst 0,5 g der letztgenannten Verbindung in 10 ml Essigsäureanhydrid, kocht die Lösung 10 Minuten unter Rückfluss und dampft sie ein. Der Rückstand wird mit Diäthyläther trituriert und aus Methanol-Essigsäureäthylester umkristallisiert. Man erhält das 1-Methyl-β-acetoxy-α-(p-acetylamino-phenylcarbamoyl)-2-pyrrolacrylnitril, welches bei 178—179° schmilzt.

Beispiel 14

Ein Gemisch von 2,1 g 1-Aethyl-β-oxo-2-pyrrolpropionitril, 25 ml Toluol und 1,6 g Triäthylamin wird unter Rühren mit 1,6 g Phenylisocyanat behandelt. Das Gemisch wird über Nacht bei Zimmertemperatur stehen gelassen, dann eingedampft und in Methanol gelöst. Die Lösung wird in 300 ml Wasser, das 5 ml 5-normaler Chlorwasserstoffsäure enthält, gegossen, der Niederschlag abgetrennt und in 5%-iger wässeriger Natriumhydroxydlösung gelöst. Die Lösung wird filtriert, das Filtrat wieder angesäuert, das feste Material abgetrennt, mit Wasser gewaschen, getrocknet und aus Aethanol umkristallisiert. Man erhält das 1-Aethyl-β-oxo-α-phenylcarbamoyl-2-pyrrolpropionitril, welches bei 144—145° schmilzt.

Der Ausgangsstoff wird wie folgt hergestellt: Eine Suspension, welche aus 9,1 g 50%igem Natriumhydrid in Mineralöl, durch Waschen mit Petroläther und Suspendieren in 50 ml Dimethylformamid hergestellt wird, wird stufenweise mit 20 g Pyrrol-2-carbonsäuremethylester in 50 ml Dimethylformamid, unter Rühren und Kühlen mit Eis versetzt. Nach Auflösung gibt man 25 ml Aethylbromid dazu und erwärmt leicht das Reaktionsgemisch, um die exothermische Reaktion einzuleiten. Wenn sich diese beruhigt, gibt man weitere 5 ml Aethylbromid dazu und lässt das Gemisch über Nacht stehen. Es wird mit Wasser behandelt und mit Diäthyläther extrahiert. Der Extrakt wird mit Wasser gewaschen, getrocknet, filtriert und eingedampft. Man erhält den öligen 1-Aethylpyrrol-2-carbonsäuremethylester.

Eine Lösung von 10 g der letztgenannten Verbindung in 25 ml Acetonitril und 25 ml Dimethylformamid wird zu einer Suspension von 5,5 g 50%igem Natriumhydrid (mit Petroläther gewaschen) in 15 ml Dimethylformamid, gegeben und das Gemisch unter Rühren 15 Minuten auf dem Dampfbad erhitzt. Man lässt es über Nacht stehen, gibt Wasser dazu, filtriert es und säuert es mit 5-normaler Chlorwasserstoffsäure an. Der Niederschlag wird abgetrennt, mit Wasser gewaschen und aus Aethanol umkristallisiert. Man erhält das 1-Aethyl-β-oxo-2-pyrrolpropionitril, welches bei 77—79° schmilzt.

Beispiel 15

Eine Lösung von 2,5 g 1-Isobutyl-β-oxo-2-pyrrolpropionitril in 30 ml Toluol und 1,6 g Triäthylamin wird mit 1,6 g Phenylisocyanat unter Rühren versetzt. Das Gemisch wird über Nacht stehen gelassen, filtriert

und der Rückstand in 5%iger wässeriger Natriumhydroxydlösung gelöst. Die Lösung wird filtriert, mit 5-normaler Chlorwasserstoffsäure angesäuert und mit einem Gemisch von Diäthyläther-Essigsäureäthylester extrahiert. Die organische Lösung wird mit Wasser gewaschen, getrocknet und eingedampft. Der Rückstand wird mit Aethanol trituriert und aus Methanol umkristallisiert. Man erhält das 1-Isobutyl-β-oxo-α-phenylcarbamoyl-2-pyrrolpropionitril, welches bei 134—136° schmilzt.

Der Ausgangsstoff wird wie folgt hergestellt: Man gibt 13 g Pyrrol-2-carbonsäuremethylester unter Rühren zu einer Suspension, welche aus 5,5 g 50%igem Natriumhydrid in Mineralöl, durch Waschen mit Petroläther und Suspendieren in 70 ml Dimethylformamid hergestellt ist. Das Gemisch wird dann mit 40 g Isobutylbromid versetzt und unter Rühren 10 Minuten auf dem Dampfbad erhitzt. Das Reaktionsgemisch wird über Nacht stehen gelassen, dann mit Wasser behandelt und mit Diäthyläther extrahiert. Der Extrakt wird mit Wasser gewaschen, getrocknet und eingedampft. Man erhält den öligen 1-Isobutylpyrrol-2-carbonsäuremethylester.

Eine Lösung von 10 g der letztgenannten Verbindung in 15 ml Acetonitril wird unter Rühren zu einer Suspension gegeben, welche aus 5 g 50%igem Natriumhydrid in Mineralöl, durch Waschen mit Petroläther und Suspendieren in 25 ml Dimethylformamid hergestellt ist. Nach dem Abklingen der brausenden Reaktion wird das Gemisch 30 Minuten auf dem Dampfbad erhitzt. Während dieser Zeit wird das Natriumhydrid aufgebracht. Man lässt das Gemisch über Nacht bei Zimmertemperatur stehen, versetzt es mit Wasser und säuert es mit 5-normaler Chlorwasserstoffsäure an. Das Gemisch wird mit Diäthyläther extrahiert, der Extrakt mit Wasser gewaschen, getrocknet und eingedampft. Man erhält das ölige 1-Isobutyl-β-oxo-2-pyrrolpropionitril.

## Beispiel 16

Eine Lösung von 1,6 g 1,3,5-Trimethyl-4-äthoxycarbonyl-β-oxo-2-pyrrolpropionitril in 30 ml trockenem Toluol und 0,8 g wasserfreiem Triäthylamin wird unter Rühren mit 0,8 g Phenylisocyanat versetzt. Das Gemisch wird 10 Minuten auf dem Dampfbad erhitzt und über Nacht bei Zimmertemperatur stehen gelassen. Dann wird es eingedampft, der Rückstand in Methanol aufgenommen und die Lösung mit 2 ml 5-normaler Chlorwasserstoffsäure und 200 ml Wasser behandelt. Die erhaltenen Kristalle werden abgetrennt, mit Wasser gewaschen, mit einem Gemisch von Methanol-Aethanol trituriert und aus Methanol umkristallisiert. Man erhält das 1,3,5-Trimethyl-4-äthoxycarbonyl-β-oxo-α-phenylcarbamoyl-2-pyrrolpropionitril, welches bei 164—166° schmilzt.

In analoger Weise werden auch das 1,3,5-Trimethyl-4-äthoxycarbonyl-β-oxo-α-(p-fluorphenylcarbamoyl)-2-pyrrolpropionitril, F. 178—179° und das 1,3,5-Trimethyl-4-äthoxycarbonyl-β-oxo-α-(p-chlorphenylcarbamoyl)-2-pyrrolpropionitril, F. 238—241° (unter Zersetzung) hergestellt.

Der Ausgangsstoff wird wie folgt hergestellt: Eine Suspension von 3,6 g 50%igem Natriumhydrid in Mineralöl (mit Petroläther gewaschen) und 50 ml Dimethylformamid wird unter Rühren mit 16 g 3,5-Dimethylpyrrol-2,4-dicarbonsäure-diäthylester in 25 ml Dimethylformamid portionweise versetzt. Das Gemisch wird nach dem Verbrauch des Natriumhydrids mit 40 ml Methyljodid versetzt und über Nacht bei Zimmertemperatur stehen gelassen. Das Gemisch wird dann mit Wasser behandelt, das erhaltene feste Material abgetrennt und zwecks Beseitigung des überschüssigen Methyljodids an der Luft getrocknet. Das feste Material wird mit Wasser gewaschen, getrocknet und aus Aethanol umkristallisiert. Man erhält den 1,3,5-Trimethylpyrrol-2,4-dicarbonsäure-diäthylester.

Eine Lösung von 13,9 g der letztgenannten Verbindung in 30 ml Acetonitril und 30 ml Dimethylformamid wird unter Rühren zu einer Suspension gegeben, welche aus 5,5 g 50%igem Natriumhydrid in Mineralöl (mit Petroläther gewaschen) und Suspendieren in 60 ml Dimethylformamid hergestellt ist. Das Gemisch wird auf dem Dampfbad 15 Minuten erwärmt und die erhaltene Lösung über Nacht stehen gelassen. Das Gemisch wird dann mit Wasser behandelt, die wässerige Lösung filtriert und mit 5-normaler Chlorwasserstoffsäure angesäuert. Der Niederschlag wird abgetrennt, mit Wasser gewaschen, mit Aethanol trituiert und aus Aethanol umkristallisiert. Man erhält das 1,3,5-Trimethyl-4-äthoxycarbonyl-β-oxo-2-pyrrolpropionitril, welches bei 105—107° schmilzt.

## Beispiel 17

Eine Lösung von 0,3 g 5-(1-Methyl-2-pyrrolyl)-4-phenylcarbamoylisoxazol in 10 ml 5%iger wässeriger Natriumhydroxydlösung und einer minimalen Menge Aethanol wird 5 Minuten auf dem Dampfbad erwärmt. Die Lösung wird dann filtriert und mit 5-normaler Chlorwasserstoffsäure angesäuert. Die erhaltenen Kristalle werden abgetrennt, mit Wasser gewaschen und mit Methanol trituriert. Man erhält das 1-Methyl-β-oxo-α-phenylcarbamoyl-2-pyrrolpropionitril, welches bei 170—172° schmilzt. Das Produkt ist mit demjenigen der Beispiele 1, 6, 9 oder 10 identisch.

Der Ausgangsstoff wird wie folgt hergestellt: Eine Lösung von 17 g 1-Methyl-pyrrol in 400 ml 1,2-Dichloräthan wird zuerst, unter Rühren und Kühlen auf 10—15° mit 28 g wasserfreiem Aluminiumchlorid portionenweise versetzt. Dann gibt man 27 g Malonsäureäthylester-chlorid in 50 ml Dichloräthan in solchen Portionen dazu, dass die Temperatur zwischen 10 und 15° bleibt. Das Gemisch wird 3,5 Stunden gerührt und dann lässt man seine Temperatur langsam auf 32° steigen. Das Reaktionsgemisch wird über Nacht bei Zimmertemperatur stehen gelassen, mit Wasser behandelt und ausgeschüttelt. Die organische Schicht wird abgetrennt, zweimal mit Wasser gewaschen, getrocknet und eingedampft. Das als Rückstand

erhaltene dunkelbraune Oel wird destilliert und die bei 105—112° und 0,25 mmHg siedende Fraktion aufgefangen. Man erhält den 1-Methyl-β-oxo-2-pyrrolpropionsäureäthylester.

Ein Gemisch von 3,6 g der letztgenannten Verbindung und 3,9 g N,N'-Diphenylformamidin wird 2,5 Stunden auf 145—165° erhitzt. Die erhaltene spröde, glasige Masse wird pulverisiert, mit Diäthyläther trituriert und aus Aethanol-Essigsäureäthylester umkristallisiert. Man erhält das 1-Methyl-β-oxo-α-anilinomethylen-2-pyrrolpropionsäureanilid, welches bei 178—180° schmilzt.

Ein Gemisch von 1,4 g der letztgenannten Verbindung, 1,1 g Hydroxylamin-hydrochlorid, 1,2 g Pyridin und 150 ml Aethanol wird 7 Stunden unter Rückfluss gekocht. Das Reaktionsgemisch wird über Nacht stehen gelassen, filtriert, eingedampft und der Rückstand mit Wasser behandelt. Das orangefarbige erhaltene Oel wird nach Stehenlassen kristallin. Der Niederschlag wird abgetrennt, mit Wasser gewaschen und aus aethanol umkristallisiert. Man erhält das 5-(1-Methyl-2-pyrrolyl)-4-phenylcarbamoylisoxazol, welches bei 100—103° schmilzt.

Beispiel 18

Eine Suspension von 1 g 1,3,5-Trimethyl-β-oxo-2-pyrrolpropionitril in 30 ml trockenem Toluol und 0,7 g wasserfreiem Triäthylamin wird unter Rühren mit 0,75 g Phenylisocyanat versetzt. Das Gemisch wird 5 Minuten auf dem Dampfbad erwärmt und über Nacht bei Zimmertemperatur stehen gelassen. Es wird filtriert, der Rückstand in Methanol aufgenommen und die Lösung mit 1-normaler Chlorwasserstoffsäure angesäuert. Der erhaltene Niederschlag wird abgetrennt, in 1-normaler wässeriger Natriumhydroxydlösung gelöst, mit 5-normaler Chlorwasserstoffsäure wieder ausgefällt und mit Wasser gewaschen. Man erhält das 1,3,5-Trimethyl-β-oxo-α-phenylcarbamoyl-2-pyrrolpropionitril, welches bei 172—174° schmilzt.

Der Ausgangsstoff wird wie folgt hergestellt: Ein Gemisch von 11,9 g 1,3,5-Trimethylpyrrol-2,4-dicarbonsäure-diäthylester und 50 ml konzentrierter Schwefelsäure wird eine Stunde auf dem Dampfbad erhitzt. Die erhaltene Lösung wird auf Eis gegossen, der Niederschlag abgetrennt und mit Wasser gewaschen. Nachher wird er in wässeriger Natriumcarbonatlösung aufgenommen, die Lösung filtriert und das Filtrat mit 5-normaler Chlorwasserstoffsäure angesäuert. Der erhaltene Niederschlag wird abgetrennt, mit Wasser gewaschen, getrocknet und aus Aethanol umkristallisiert. Man erhält die 1,3,5-Trimethyl-2-carbäthoxypyrrol-4-carbonsäure, welche unter Zersetzung bei 197—198° schmilzt.

Man erhitzt 15 Minuten unter Rückfluss bei 235—240° 7,5 g der letztgenannten Verbindung und kühlt sie dann ab. Der Rückstand wird in Petroläther aufgenommen, die Lösung filtriert und eingedampft. Man erhält den 1,3,5-Trimethylpyrrol-2-carbonsäureäthylester, der bei 38—40° schmilzt. (Das Produkt ist im US-Patent 2,479,972 mit einem Siedepunkt von 102—108°/3—4 mmHg beschrieben).

Eine Lösung von 4 g der letztgenannten Verbindung in 10 ml Acetonitril wird unter Rühren zu einer Suspension gegeben, welche aus 2 g 50%igem Natriumhydrid in Mineralöl (gewaschen mit Petroläther) und Suspendieren in 15 ml Dimethylformamid hergestellt ist. Das Gemisch wird 25 Minuten auf dem Dampfbad erhitzt und 3 Stunden bei Zimmertemperatur stehen gelassen. Das Gemisch wird mit Wasser versetzt, die wässerige Lösung mit Diäthyläther extrahiert und mit 5-normaler Chlorwasserstoffsäure angesäuert. Die Kristalle werden abgetrennt, mit Wasser gewaschen, getrocknet und aus Aethanol unter Zugabe von Aktivkohle umkristallisiert. Man erhält das 1,3,5-Trimethyl-β-oxo-2-pyrrolpropionitril, welches bei 106—107° schmilzt.

In analoger Weise wird auch das 1,3,5-Trimethyl-β-oxo-α-(p-fluorphenylcarbamoyl)-2-pyrrolpropionitril erhalten, welches nach Umkristallisation aus Methanol-Aethanol (1:1) bei 184—186° schmilzt.

Beispiel 19

Eine Suspension von 1 g 1,2,5-Trimethyl-β-oxo-3-pyrrolpropionitril in 40 ml Toluol und 0,7 g Triäthylamin wird mit 0,75 g Phenylisocyanat behandelt und 5 Minuten auf dem Dampfbad, bis zur Auflösung des festen Materials erwärmt. Das Gemisch wird über Nacht stehen gelassen, der erhaltene Niederschlag abgetrennt, in Methanol aufgenommen und die Lösung zu einem Gemisch von 3 ml 5-normaler Chlorwasserstoffsäure und 250 ml Wasser gegeben. Das Rohprodukt wird abgetrennt, mit Wasser gewaschen, in 5%iger wässeriger Natriumhydroxydlösung gelöst, filtriert und das alkalische Filtrat mit 5-normaler Chlorwasserstoffsäure angesäuert. Der Niederschlag wird abgetrennt, mit Wasser gewaschen, an der Luft getrocknet und aus Aethanol umkristallisiert. Man erhält das 1,2,5-Trimethyl-β-oxo-α-phenylcarbamoyl-3-pyrrolpropionitril, welches bei 158—160° schmilzt.

In analoger Weise wird auch das rohe 1,2,5-Trimethyl-β-oxo-α-(p-fluorphenylcarbamoyl)-3-pyrrolpropionitril erhalten. Es wird in wässeriger Natriumhydrogencarbonatlösung gelöst, mit 5-normaler Chlorwasserstoffsäure ausgefällt und aus Aethanol umkristallisiert. F. 171—172°.

Der Ausgangsstoff wird wie folgt hergestellt: Eine Lösung von 7,6 g 1,2,5-Trimethylpyrrol-3-carbonsäure-äthylester [Ber. *56*, 2374 (1923), Schmelzpunkt nach Umkristallisation aus Methanol 65—66°] in 20 ml Acetonitril wird zu einer Suspension von 4 g 50%igem Natriumhydrid in Mineralöl und 11 ml Dimethylformamid gegeben. Das Gemisch wird unter Rühren 20 Minuten auf dem Dampfbad erhitzt und die erhaltene Suspension innerhalb 90 Minuten auf Zimmertemperatur abkühlen gelassen. Die Suspension wird in 200 ml Eiswasser gegossen, die alkalische wässerige Lösung filtriert und das Filtrat unter Kühlung mit Eis, mit 18%iger Chlorwasserstoffsäure angesäuert. Der Niederschlag wird abgetrennt, mit Wasser gewaschen, und an der Luft getrocknet, mit Diäthyläther trituriert und aus Methanol umkristallisiert. Man erhält das 1,2,5-Trimethyl-β-oxo-3-pyrrolpropionitril, welches bei 140—141° schmilzt.

Beispiel 20

Ausgehend von äquivalenten Mengen entsprechender Ausgangsstoffe werden auch gemäss den Verfahren der vorhergehenden Beispiele, insbesondere gemäss den Beispielen 1, 4, 8 und 10, die folgenden Verbindungen der Formel II, worin $R_2 = R_3 = H$, hergestellt:

| No. | $R_1$ | $R_4$ | $R_5$ | Umkristallisiert aus | F.°C |
|---|---|---|---|---|---|
| 1 | $CH_3$ | 2-$CH_3$ | 6-$CH_3$ | Aethanol | 170—171 |
| 2 | $CH_3$ | 3-F | H | Aethylacetat | 191—192 |
| 3 | $CH_3$ | 3-Cl | H | Aethylacetat | 192—193 |
| 4 | $CH_3$ | 4-Cl | H | Aethylacetat | 205—207 |
| 5 | $CH_3$ | 3-$CF_3$ | H | Methanol | 185—186 |
| 6 | $CH_3$ | 2-F | 4-F | Methanol | 136—138 |
| 7 | $CH_3$ | 4-F | 3-Cl | Aethylacetat | 215—216 |
| 8 | $CH_3$ | 2-Cl | 4-Cl | Aethylacetat | 160—162 |
| 9 | $CH_3$ | 3-Cl | 4-Cl | Dimethylformamid | 217—219 |
| 10 | Benzyl | H | H | Aethylacetat | 188—189 |
| 11 | Benzyl | 4-F | H | Aethylacetat | 203—204 |
| 12 | $C_2H_5$ | 4-F | H | Methanol | 156—157 |
| 13 | $C_2H_5$ | 4-Cl | H | Aethanol | 160—161 |
| 14 | i-Butyl | 4-F | H | Methanol | 162—163 |

Beispiel 21

Herstellung von 10 000 Tabletten mit einem Gehalt von je 100 mg der aktiven Substanz:

*Bestandteile:*

| | |
|---|---|
| Tris-hydroxyäthylammonium-1-methyl-β-oxo-α-phenylcarbamoyl-2-pyrrolpropionitril | 1000 g |
| Milchzucker | 2535 g |
| Maisstärke | 125 g |
| Polyäthylenglykol 6000 | 150 g |
| Talkpulver | 150 g |
| Magnesiumstearat | 40 g |
| Gereinigtes Wasser | q.s. |

*Verfahren:*

Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 65 ml Wasser suspendiert und die Suspension zur siedenden Lösung von Polyäthylenglykol in 260 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten mit 10,3 mm Durchmesser, welche eine Bruchrille aufweisen, gepresst.

In analoger Weise werden Tabletten, welche eine andere, in den vorhergehenden Beispielen illustrierte Verbindung enthalten, hergestellt.

Beispiel 22

Herstellung von 1000 Kapseln mit einem Gehalt von je 25 mg der aktiven Substanz:

*Verfahren:*

| | |
|---|---|
| 1-Methyl-β-oxo-α-(p-fluorphenylcarbamoyl)-2-pyrrolpropionitril | 25 g |
| Milchzucker | 207 g |
| Maisstärke | 80 g |
| Magnesiumstearat | 3 g |

*Verfahren:*

Sämtliche pulverigen Bestandteile werden mit einem Sieb von 0,6 mm Maschenweite gesiebt. Dann wird der Wirkstoff zuerst mit Magnesiumstearat und darauffolgend mit Milchzucker und Stärke in einem geeigneten Mischer homogenisiert. Kapseln Nr. 2 werden mit je 315 mg des Gemisches mit einer Füllmaschine gefüllt.

In analoger Weise werden Kapseln, welche 25—100 mg von anderen genannten und illustrierten Verbindungen, insbesondere solchen der Formel II, oder von ihren Alkalimetall-, Zink-, Tri-niederalkylammonium- oder Tris-hydroxyäthylammoniumsalzen enthalten, hergestellt.

**Patentansprüche für die Vertragsstaaten: BE CH DE FR GB IT LI LU NL SE**

1. Substituierte β-Oxo-α-phenylcarbamoyl-pyrrolpropionitrile der allgemeinen Formel I

CN
|
Pl—CO—CH—CONH—Ph (I),

worin Pl einen 2- oder 3-Pyrrolylrest bedeutet, der in 1-Stellung durch $C_1$—$C_7$-Alkyl oder Ph-$C_1$—$C_7$-Alkyl substituiert ist und in den übrigbleibenden drei Stellungen unsubstituiert oder durch eine bis drei, gleiche oder verschiedene $C_1$—$C_7$-Alkylgruppen und/oder eine Carboxy- oder Carbo-$C_1$—$C_7$-alkoxygruppe substituiert ist; und Ph Phenyl bedeutet, das unsubstituiert oder durch einen bis drei, gleiche oder verschiedene Substituenten der Gruppe $C_1$—$C_7$-Alkyl, $C_1$—$C_7$-Alkoxy, $C_1$—$C_7$-alkylthio, Hydroxy, Halogen, Trifluormethyl, Nitro, Amino oder $C_1$—$C_7$-Alkanoylamino substituiert ist; diese Verbindungen in ihrer Enolform, ihre Enol-$C_1$—$C_7$-alkyläther oder Enol-$C_1$—$C_7$-alkanoylester, oder ihre Salze mit Basen.

2. Verbindungen nach Anspruch 1, Formel I, worin Pl 1-($C_1$—$C_7$-Alkyl oder Ph-$C_1$—$C_7$-Alkyl)-2- oder -3-pyrrolyl bedeutet, welches in den übrigbleibenden Stellungen unsubstituiert oder durch eine oder zwei $C_1$—$C_7$-Alkylgruppen, oder eine Carboxy- oder Carbo-$C_1$—$C_7$-alkoxygruppe substituiert ist, Ph für Phenyl steht, das unsubstituiert oder durch einen oder zwei, gleiche oder verschiedene Substituenten der Gruppe $C_1$—$C_7$-Alkyl, $C_1$—$C_7$-Alkoxy, $C_1$—$C_7$-Alkylthio, Hydroxy, Halogen, Trifluormethyl, Nitro, Amino und $C_1$—$C_7$-Alkanoylamino substituiert ist, ihre Enol-$C_1$—$C_7$-alkyläther oder Enol-$C_1$—$C_7$-alkanoylester, oder ihre salze mit Basen.

3. Verbindungen nach Anspruch 1, Formel I, worin Pl einen 2- oder 3-Pyrrolylrest bedeutet, der in 1-Stellung durch $C_1$—$C_7$-Alkyl oder Ph-$C_1$—$C_7$-Alkyl substituiert ist und in den übrigbleibenden drei Stellungen unsubstituiert oder durch eine bis drei, gleiche oder verschiedene $C_1$—$C_7$-Alkylgruppen substituiert ist; und Ph Phenyl bedeutet, das unsubstituiert oder durch einen bis drei, gleiche oder verschiedene Substituenten der Gruppe $C_1$—$C_7$-Alkyl, $C_1$—$C_7$-Alkoxy, $C_1$—$C_7$-Alkylthio, Hydroxy, Halogen, Trifluormethyl, Nitro oder Amino substituiert ist, ihre Enol-$C_1$—$C_7$-alkyläther oder ihre Salze mit Basen.

4. Verbindungen nach Anspruch 1, welche der allgemeinen Formel II

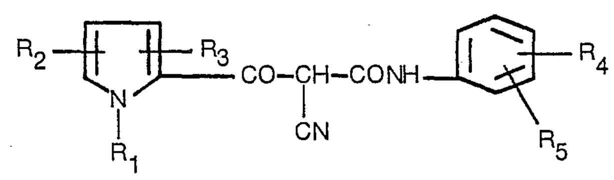

(II),

entsprechen, worin $R_1$ $C_1$—$C_7$-Alkyl bedeutet, jedes der Symbole $R_2$ und $R_3$ für Wasserstoff oder $C_1$—$C_7$-Alkyl steht, und jedes der Symbole $R_4$ und $R_5$ Wasserstoff, $C_1$—$C_7$-Alkyl, $C_1$—$C_7$-Alkoxy, Hydroxy, Halogen oder Trifluormethyl bedeutet, oder ihre Salze mit Basen.

5. Verbindungen nach Anspruch 1, welche der im Anspruch 4 gezeigten Formel II entsprechen, worin $R_1$ Alkyl mit höchstens 4 Kohlenstoffatomen bedeutet, jedes der Symbole $R_2$ und $R_3$ für Wasserstoff steht, und jedes der Symbole $R_4$ und $R_5$ Wasserstoff, Alkyl oder Alkoxy, jeweils mit höchstens 4 Kohlenstoffatomen, Fluor, Chlor oder Trifluormethyl bedeutet, oder ihre Salze mit Basen.

6. Verbindungen nach Anspruch 1, welche der im Anspruch 4 gezeigten Formel II entsprechen, worin $R_1$ Methyl bedeutet, jedes der Symbole $R_2$ und $R_3$ für Wasserstoff steht, und jedes der Symbole $R_4$ und $R_5$ Wasserstoff, Methyl, Methoxy, Fluor, Chlor oder Trifluormethyl bedeutet, oder ihre Salze mit Basen.

7. 1-Methyl-β-oxo-α-phenylcarbamoyl-2-pyrrolpropionitril und seine Salze mit Basen.

8. 1-Methyl-β-oxo-α-(p-fluorphenylcarbamoyl)-2-pyrrolpropionitril und seine Salze mit Basen.

9. Antiinflammatorisch und antiarthritisch wirksame Verbindungen gemäss Anspruch 1.

10. Antiinflammatorisch und antiarthritisch wirksame Verbindungen gemäss Anspruch 3.

11. Pharmazeutische Präparate enthaltend Verbindungen gemäss einem der Ansprüche 1, 2 und 9 wobei von den Salzen nur solche in Frage kommen die therapeutisch verwendbar sind.

12. Pharmazeutische Präparate enthaltend Verbindungen gemäss einem der Ansprüche 3 bis 8 und 10 wobei von den Salzen nur solche in Frage kommen die therapeutisch verwendbar sind.

13. Verwendung von Verbindungen der Ansprüche 1, 2 und 9 zur Herstellung von pharmazeutischen Präparaten.

14. Verwendung von Verbindungen der Ansprüche 3 bis 8 und 10 zur Herstellung von pharmazeutischen Präparaten.

15. Die Verbindungen der Ansprüche 1, 2 und 9 zur Anwendung in einem Verfahren zur therapeutischen Behandlung des menschlichen oder tierischen Körpers.

16. Die Verbindungen der Ansprüche 3 bis 8 und 10 zur Anwendung in einem Verfahren zur therapeutischen Behandlung des menschlichen oder tierischen Körpers.

17. Verfahren zur Herstellung von im Anspruch 1 definierten substituierten β-Oxo-α-phenylcarbamoylpyrrolpropionitrilen, dadurch gekennzeichnet, dass man

a) Verbindung der Formeln

$$Pl—CO—CH_2—CN \text{ und } OC{=}N—Ph$$

addiert, oder

b) Verbindungen der Formeln

Pl—CO—CH—COX
|
CN

und H—NH—Ph,

worin X $C_1$—$C_7$-Alkoxy, $C_1$—$C_7$-Alkanoyloxy oder Halogen bedeutet, kondensiert oder

c) Verbindungen der Formeln

Pl—Y und $CH_2$—CONH—Ph
|
CN

worin Y $C_1$—$C_7$-Alkoxycarbonyl, Halogencarbonyl oder Cyan bedeutet, kondensiert und die erhaltenen Imine hydrolysiert oder

d) Verbindung der Formel

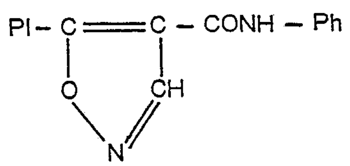

mit einer starken Base isomerisiert, und, wenn erwünscht, eine erhaltene Verbindung der Formel I in eine andere Verbindung der Erfindung umwandelt, und/oder, wenn erwünscht, ein erhaltenes Enol in ein Enol-$C_1$—$C_7$-alkyläther, oder Enol-$C_1$—$C_7$-alkanoylester überführt, und/oder, wenn erwünscht, ein erhaltenes Enol in ein Salz mit einer Base oder ein erhaltenes Enolsalz in das freie Enol oder in ein anderes Salz mit einer Base überführt, und/oder, wenn erwünscht, ein erhaltenes Gemisch von Isomeren in die einzelnen Isomeren auftrennt.

18. Die nach dem Verfahren des Anspruchs 17 erhältlichen Verbindungen.

**Patentanspüche für den Vertragsstaat: AT**

1. Verfahren zur Herstellung von substituierten β-Oxo-α-phenylcarbamoyl-pyrrolpropionitrilen der allgemeinen Formel I

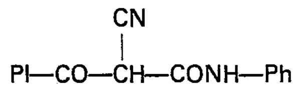 (I),

worin Pl einen 2- oder 3-Pyrrolylrest bedeutet, der in 1-Stellung durch $C_1$—$C_7$-Alkyl oder Ph-$C_1$—$C_7$-Alkyl substituiert ist und in den übrigbleibenden drei Stellungen unsubstituiert oder durch eine bis drei, gleiche oder verschiedene $C_1$—$C_7$-Alkylgruppen und/oder eine Carboxy- oder Carbo-$C_1$—$C_7$-alkoxygruppe substituiert ist; und Ph Phenyl bedeutet, das unsubstituiert oder durch einen bis drei, gleiche oder verschiedene Substituenten der Gruppe $C_1$—$C_7$-Alkyl, $C_1$—$C_7$-Alkoxy, $C_1$—$C_7$-alkylthio, Hydroxy, Halogen, Trifluormethyl, Nitro, Amino oder $C_1$—$C_7$-Alkanoylamino substituiert ist, von diesen Verbindungen in ihrer Enolform, ihren Enol-$C_1$—$C_7$-Alkyläthern oder Enol-$C_1$—$C_7$-Alkanoylester, oder ihren Salzen mit Basen, dadurch gekennzeichnet, dass man

a) Verbindungen der Formeln

Pl—CO—$CH_2$—CN und OC=N—Ph

addiert,

b) Verbindungen der Formeln

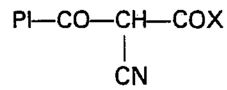

und H—NH—Ph,

worin X $C_1$—$C_7$-Alkoxy, $C_1$—$C_7$-Alkanoyloxy oder Halogen bedeutet, kondensiert oder

c) Verbindungen der Formeln

Pl—Y und 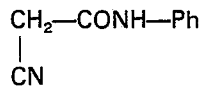

worin Y $C_1$—$C_7$-Alkoxycarbonyl, Halogencarbonyl oder Cyan bedeutet, kondensiert und die erhaltenen Imine hydrolysiert oder

d) Verbindung der Formel

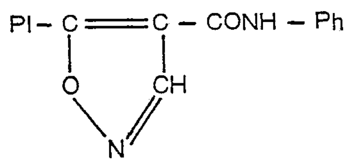

mit einer starken Base isomerisiert, und, wenn erwünscht, eine erhaltene Verbindung der Formel I in eine andere Verbindung der Erfindung umwandelt, und/oder, wenn erwünscht, ein erhaltenes Enol in ein Enol-$C_1$—$C_7$-alkyläther, oder Enol-$C_1$—$C_7$-alkanoylester überführt, und/oder, wenn erwünscht, ein erhaltenes Enol in ein Salz mit einer Base oder ein erhaltenes Enolsalz in das freie Enol oder in ein anderes Salz mit einer Base überführt, und/oder, wenn erwünscht, ein erhaltenes Gemisch von Isomeren in die einzelnen Isomeren auftrennt.

2. Verfahren nach Anspruch 1, dadurch gekennzeichnet, dass man die Reaktion a) in Gegenwart einer Base oder bei erhöhter Temperatur durchführt.

3. Verfahren nach Anspruch 1, dadurch gekennzeichnet, dass man die Reaktion b) bei erhöhter Temperatur durchführt.

4. Verfahren nach Anspruch 1, dadurch gekennzeichnet, dass man die Reaktion c) in Gegenwart eines Alkalimetall-alkoxids oder- hydrids durchführt.

5. Verfahren nach Anspruch 1, dadurch gekennzeichnet, dass man die Reaktion d) in Gegenwart einer starken anorganischen oder organischen Base durchführt.

6. Verfahren nach Anspruch 1, dadurch gekennzeichnet, dass man in einer erhaltenen Verbindung, welche im Rest Ph als Substituenten eine Nitrogruppe aufweist, diese zu der Aminogruppe reduziert.

7. Verfahren nach einem der Ansprüche 1 bis 6, dadurch gekennzeichnet, dass man in einer erhaltenen Verbindung, welche im Rest Ph als Substituenten die Aminogruppe aufweist, diese zu einer $C_1$—$C_7$-Alkanoylaminogruppe acyliert.

8. Verfahren nach einem der Ansprüche 1 bis 7, dadurch gekennzeichnet, dass man Verbindungen der im Anspruch 1 gezeigten Formel I, worin Pl 1-($C_1$—$C_7$-Alkyl oder Ph-$C_1$—$C_7$-Alkyl)-2- oder -3-pyrrolyl bedeutet, welches in den übrigbleibenden Stellungen unsubstituiert oder durch eine oder zwei $C_1$—$C_7$-Alkylgruppen, oder eine Carboxy- oder Carbo-$C_1$—$C_7$-alkoxygruppe substituiert ist, Ph für Phenyl steht, das unsubstituiert oder durch einen oder zwei, gleiche oder verschiedene Substituenten der Gruppe $C_1$—$C_7$-Alkyl, $C_1$—$C_7$-Alkoxy, $C_1$—$C_7$-Alkylthio, Hydroxy, Halogen, Trifluormethyl, Nitro, Amino und $C_1$—$C_7$-

Alkanoylamino substituiert ist, ihre Enol-$C_1$—$C_7$-alkyläther oder Enol-$C_1$—$C_7$-alkanoylester, oder ihre Salze mit Basen herstellt.

9. Verfahren nach Anspruch 1 zur Herstellung von neuen substituierten β-Oxo-α-phenylcarbamoyl-pyrrolpropionitrilen der im Anspruch 1 gezeigten Formel I, worin Pl einen 2- oder 3-Pyrrolylrest bedeutet, der in 1-Stellung durch $C_1$—$C_7$-Alkyl oder Ph-$C_1$—$C_7$-Alkyl substituiert ist und in den übrigbleibenden drei Stellungen unsubstituiert oder durch eine bis drei, gleiche oder verschiedene $C_1$—$C_7$-Alkylgruppen substituiert ist; und Ph Phenyl bedeutet, das unsubstituiert oder durch einen bis drei, gleiche oder verschiedene Substituenten der Gruppe $C_1$—$C_7$-Alkyl, $C_1$—$C_7$-Alkoxy, $C_1$—$C_7$-Alkylthio, Hydroxy, Halogen, Trifluormethyl, Nitro oder Amino substituiert ist; ihren Enol-$C_1$—$C_7$-Alkyläthern oder ihren Salzen mit Basen, dadurch gekennzeichnet, dass man

a) Ausgangsstoffe der in Anspruch 1 angegebenen Formeln addiert, oder

b) Ausgangsstoffe der im Anspruch 1 angegebenen Formeln kondensiert oder

c) Ausgangsstoffe der im Anspruch 1 angegebenen Formeln kondensiert und die erhaltenen Imine hydrolysiert oder

d) Verbindungen der im Anspruch 1 angegebenen Formel mit einer starken Base isomerisiert, und, wenn erwünscht, eine erhaltene Verbindung der Formel I in eine andere Verbindung der Erfindung umwandelt, und/oder, wenn erwünscht, ein erhaltenes Enol in ein Enol-$C_1$—$C_7$-alkyläther überführt, und/oder, wenn erwünscht, ein erhaltenes Enol in ein Salz mit einer Base oder ein erhaltenes Enolsalz in das freie Enol oder in ein anderes Salz mit einer Base überführt, und/oder, wenn erwünscht, ein erhaltenes Gemisch von Isomeren in die einzelnen Isomeren auftrennt.

10. Verfahren nach Anspruch 9, dadurch gekennzeichnet, dass man die Reaktionen a), b), c) und d) gemäss den Ansprüchen 2, 3, 4 bzw. 5 durchführt.

11. Verfahren nach einem der Ansprüche 9 und 10, dadurch gekennzeichnet, dass man in einer erhaltenen Verbindung, welche im Rest Ph als Substituenten eine Nitrogruppe aufweist, diese zu der Aminogruppe reduziert.

12. Verfahren nach einem der Ansprüche 9 und 10, dadurch gekennzeichnet, dass man Verbindungen der allgemeinen Formel II

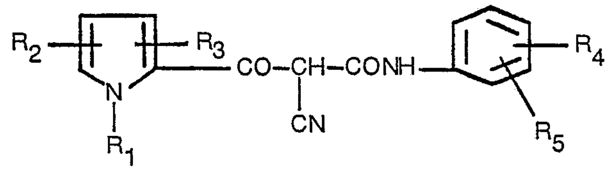 (II),

worin $R_1$ $C_1$—$C_7$-Alkyl bedeutet, jedes der Symbole $R_2$ und $R_3$ für Wasserstoff oder $C_1$—$C_7$-Alkyl steht, und jedes der Symbole $R_4$ und $R_5$ Wasserstoff, $C_1$—$C_7$-Alkyl, $C_1$—$C_7$-Alkoxy, Hydroxy, Halogen oder Trifluormethyl bedeutet, oder ihre Salze mit Basen herstellt.

13. Verfahren nach einem der Ansprüche 9 und 10, dadurch gekennzeichnet, dass man Verbindungen der im Anspruch 12 gezeigten Formel II, worin $R_1$ Alkyl mit höchstens 4 Kohlenstoffatomen bedeutet, jedes der Symbole $R_2$ und $R_3$ für Wasserstoff steht, und jedes der Symbole $R_4$ und $R_5$ Wasserstoff, Alkyl oder Alkoxy, jeweils mit höchstens 4 Kohlenstoffatomen, Fluor, Chlor oder Trifluormethyl bedeutet, oder ihre Salze mit Basen herstellt.

14. Verfahren nach einem der Ansprüche 9 und 10, dadurch gekennzeichnet, dass man Verbindungen der im Anspruch 12 gezeigten Formel II worin $R_1$ Methyl bedeutet, jedes der Symbole $R_2$ und $R_3$ für Wasserstoff steht, und jedes der Symbole $R_4$ und $R_5$ Wasserstoff, Methyl, Methoxy, Fluor, Chlor oder Trifluormethyl bedeutet, oder ihre Salze mit Basen herstellt.

15. Verfahren nach einem der Ansprüche 9 und 10, dadurch gekennzeichnet, dass man 1-Methyl-β-oxo-α-phenylcarbamoyl-2-pyrrolpropionitril und seine Salze mit Basen herstellt.

16. Verfahren nach einem der Ansprüche 9 und 10, dadurch gekennzeichnet, dass man 1-Methyl-β-oxo-α-(p-fluorphenylcarbamoyl)-2-pyrrolpropionitril und seine Salze mit Basen herstellt.

17. Verfahren zur Herstellung eines pharmazeutischen Präparates, gekennzeichnet, durch die Verarbeitung eines erfindungsgemässen Wirkstoffs nach einem der Ansprüche 1 bis 8, mit einem pharmazeutischen Trägermaterial.

18. Verfahren zur Herstellung eines pharmazeutischen Präparates, gekennzeichnet, durch die Verarbeitung eines erfindungsgemässen Wirkstoffs nach einem der Ansprüche 9 bis 16, mit einem pharmazeutischen Trägermaterial.

**Claims for the Contracting States: DE GB FR CH LI IT NL BE SE LU**

1. A substituted β-oxo-α-phenylcarbamoylpyrrolepropionitrile of the general formula I

$$\begin{array}{c} CN \\ | \\ Pl—CO—CH—CONH—Ph \end{array} \quad (I),$$

wherein Pl is 2- or 3-pyrrolyl substituted in the 1-position by $C_1$—$C_7$alkyl or Ph-$C_1$—$C_7$alkyl, and unsubstituted in the remaining 3 positions or substituted therein by one to three identical or different $C_1$—$C_7$alkyl groups and/or by one carboxy or $C_1$—$C_7$carbalkoxy group; and Ph is phenyl which is unsubstituted or substituted by one to three identical or different members selected from $C_1$—$C_7$alkyl, $C_1$—$C_7$alkoxy, $C_1$—$C_7$alkylthio, hydroxy, halogen, trifluoromethyl, nitro, amino or $C_1$—$C_7$alkanoylamino; this compound in its enol form, a $C_1$—$C_7$alkyl enol ether or a $C_1$—$C_7$alkanoyl enol ester thereof, or a salt thereof with a base.

2. A compound according to claim 1 of the formula I, wherein Pl is 1-($C_1$—$C_7$alkyl or Ph-$C_1$—$C_7$alkyl)-2- or -3-pyrrolyl which is unsubstituted in the remaining positions or substituted therein by one or two $C_1$—$C_7$alkyl groups, or by one carboxy or $C_1$—$C_7$carbalkoxy group; Ph is phenyl or phenyl which is substituted by one or two identical or different members selected from $C_1$—$C_7$alkyl, $C_1$—$C_7$alkoxy, $C_1$—$C_7$alkylthio, hydroxy, halogen, trifluoromethyl, nitro, amino and $C_1$—$C_7$alkanoylamino; or a $C_1$—$C_7$alkyl enol ether or a $C_1$—$C_7$alkanoyl enol ester thereof, or a salt thereof with a base.

3. A compound according to claim 1 of the formula I, wherein Pl is 2- or 3-pyrrolyl substituted in the 1-position by $C_1$—$C_7$alkyl or Ph-$C_1$—$C_7$alkyl, and unsubstituted in the remaining 3 positions or substituted therein by one to three identical or different $C_1$—$C_7$alkyl groups; and Ph is phenyl which is unsubstituted or substituted by one to three identical or different members selected from $C_1$—$C_7$alkyl, $C_1$—$C_7$alkoxy, $C_1$—$C_7$alkylthio, hydroxy, halogen, trifluoromethyl, nitro or amino; or a $C_1$—$C_7$alkyl enol ether thereof, or a salt thereof with a base.

4. A compound according to claim 1 of the general formula II

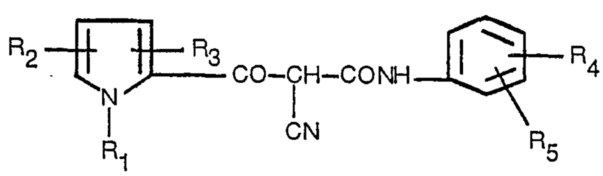

(II),

wherein $R_1$ is $C_1$—$C_7$alkyl; each of $R_2$ and $R_3$ is hydrogen or $C_1$—$C_7$alkyl; and each of $R_4$ and $R_5$ is hydrogen, $C_1$—$C_7$alkyl, $C_1$—$C_7$alkoxy, hydroxy, halogen or trifluoromethyl; or a salt thereof with a base.

5. A compound according to claim 1 of the formula II as indicated in claim 4, wherein $R_1$ is alkyl of not more than 4 carbon atoms; each of $R_2$ and $R_3$ is hydrogen; and each of $R_4$ and $R_5$ is hydrogen, alkyl or alkoxy of not more than 4 carbon atoms each, fluoro, chloro or trifluoromethyl, or a salt thereof with a base.

6. A compound according to claim 1 of the formula II as indicated in claim 4, wherein $R_1$ is methyl; each of $R_2$ and $R_3$ is hydrogen; and each of $R_4$ and $R_5$ is hydrogen, methyl, methoxy, fluoro, chloro or trifluoromethyl, or a salt thereof with a base.

7. 1-Methyl-β-oxo-α-phenylcarbamoyl-2-pyrrolepropionitrile, or a salt thereof with a base.

8. 1-Methyl-β-oxo-α-(p-fluorophenylcarbamoyl)-2-pyrrolepropionitrile, or a salt thereof with a base.

9. A compound according to claim 1 having antiinflammatory and antiarthritic activity.

10. A compound according to claim 3 having antiinflammatory and antiarthritic activity.

11. A pharmaceutical composition containing a compound as claimed in any one of claims 1, 2 or 9, or a therapeutically acceptable salt thereof.

12. A pharmaceutical composition containing a compound as claimed in any one of claims 3 to 8 or 10, or a therapeutically acceptable salt thereof.

13. Use of a compound as claimed in any one of claims 1, 2 or 9 for the preparation of a pharmaceutical composition.

14. Use of a compound as claimed in any one of claims 3 to 8 or 10 for the preparation of a pharmaceutical composition.

15. Use of a compound as claimed in any one of claims 1, 2 or 9 in a therapeutic method of treating the human or animal body.

16. Use of a compound as claimed in any one of claims 3 to 8 or 10 in a therapeutic method of treating the human or animal body.

17. A process for the preparation of a substituted β-oxo-α-phenylcarbamoylpyrrolepropionitrile as defined in claim 1, which comprises

a) adding compounds of formulae

$$Pl—CO—CH_2—CN + OC{=}N—Ph$$

b) condensing compounds of the formulae

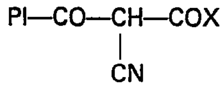

and R—NH—Ph,
wherein X is $C_1$—$C_7$alkoxy, $C_1$—$C_7$alkanoyloxy or halogen, or

c) condensing compounds of formulae

Pl—Y and 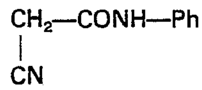

wherein Y is $C_1$—$C_7$alkoxycarbonyl, halocarbonyl or cyano and hydrolysing the resultant imines, or

d) isomerizing compounds of the formula

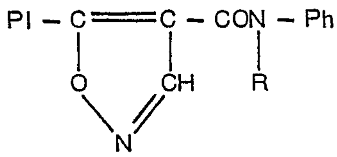

with a strong base and, if desired, converting a resultant compound of formula I into another compound of the invention, and/or, if desired, converting a resultant enol into a $C_1$—$C_7$alkyl enol ether or a $C_1$—$C_7$alkanoyl enol ester, and/or, if desired, converting a resultant enol into a salt with a base, or a resultant enol salt into the free enol or into another salt with a base, and/or, if desired, resolving a mixture of isomers obtained into the individual isomers.

18. A compound obtainable according to the process of claim 17.

**Claims for the Contracting State: AT**

1. A process for the preparation of a substituted β-oxo-α-phenylcarbamoylpyrrolepropionitrile of the general formula I

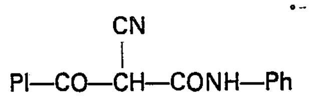

(I)

wherein Pl is 2- or 3-pyrrolyl substituted in the 1-position by $C_1$—$C_7$alkyl or Ph-$C_1$—$C_7$alkyl, and unsubstituted in the remaining 3 positions or substituted therein by one to three identical or different $C_1$—$C_7$alkyl groups and/or by one carboxy or $C_1$—$C_7$carbalkoxy group; and Ph is phenyl which is unsubstituted or substituted by one to three identical or different members selected from $C_1$—$C_7$alkyl, $C_1$—$C_7$alkoxy, $C_1$—$C_7$alkylthio, hydroxy, halogen, trifluoromethyl, nitro, amino or $C_1$—$C_7$alkanoylamino; of this compound in its enol form, a $C_1$—$C_7$alkyl enol ether or a $C_1$—$C_7$alkanoyl enol ester thereof, or a salt thereof with a base, which process comprises

a) adding compounds of formulae

Pl—CO—$CH_2$—CN + OC=N—Ph

b) condensing compounds of the formulae

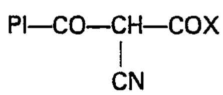

and R—NH—Ph,
wherein X is $C_1$—$C_7$alkoxy, $C_1$—$C_7$alkanoyloxy or halogen, or

c) condensing compounds of formulae

Pl—Y and 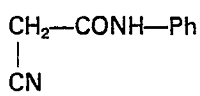

wherein Y is $C_1$—$C_7$alkoxycarbonyl, halocarbonyl or cyano and hydrolysing the resultant imines, or

d) isomerizing compounds of the formula

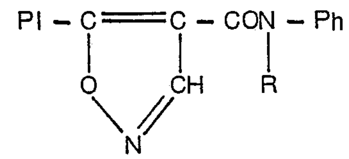

with a strong base and, if desired, converting a resultant compound of formula I into another compound of the invention, and/or, if desired, converting a resultant enol into a $C_1$—$C_7$alkyl enol ether or a $C_1$—$C_7$alkanoyl enol ester, and/or, if desired, converting a resultant enol into a salt with a base, or a resultant enol salt into the free enol or into another salt with a base, and/or, if desired, resolving a mixture of isomers obtained into the individual isomers.

2. A process according to claim 1, wherein the reaction a) is carried out in the presence of a base or at elevated temperature.

3. A process according to claim 1, wherein the reaction b) is carried out at elevated temperature.

4. A process according to claim 1, wherein the reaction c) is carried out in the presence of an alkali metal alkoxide or hydride.

5. A process according to claim 1, wherein the reaction d) is carried out in the presence of a strong inorganic or organic base.

6. A process according to claim 1, wherein in a resultant compound having a nitro group as substituent in the Ph radical, said group is reduced to the amino group.

7. A process according to any one of claims 1 to 6, wherein in a resultant compound having the amino group as substituent in the Ph radical, said group is acylated to a $C_1$—$C_7$alkanoylamino group.

8. A process according to any one of claims 1 to 7, which process comprises preparing a compound of the formula I as indicated in claim 1, wherein Pl is a 1-($C_1$—$C_7$alkyl or Ph-$C_1$—$C_7$alkyl)-2- or -3-pyrrolyl which is unsubstituted in the remaining positions or substituted therein by one or two $C_1$—$C_7$alkyl groups, or by one carboxy or $C_1$—$C_7$carbalkoxy group; Ph is phenyl or phenyl which is substituted by one or two identical or different members selected from $C_1$—$C_7$alkyl, $C_1$—$C_7$alkoxy, $C_1$—$C_7$alkylthio, hydroxy, halogen, trifluoromethyl, nitro, amino and $C_1$—$C_7$alkanoylamino; or a $C_1$—$C_7$alkyl enol ether or a $C_1$—$C_7$alkanoyl enol ester thereof, or a salt thereof with a base.

9. A process according to claim 1 for the preparation of a novel substituted β-oxo-α-phenylcarbamoyl-pyrrolepropionitrile of the formule I as indicated in claim 1, wherein Pl is 2- or 3-pyrrolyl substituted in the 1-position by $C_1$—$C_7$alkyl or Ph-$C_1$—$C_7$alkyl, and unsubstituted in the remaining 3 positions or substituted therein by one to three identical or different $C_1$—$C_7$alkyl groups; and Ph is phenyl which is unsubstituted or substituted by one to three identical or different members selected from $C_1$—$C_7$alkyl, $C_1$—$C_7$alkoxy, $C_1$—$C_7$alkylthio, hydroxy, halogen, trifluoromethyl, nitro or amino; or a $C_1$—$C_7$alkyl enol ether thereof, or a salt thereof with a base, which process comprises

a) adding starting materials of the formulae as indicated in claim 1, or

b) condensing starting materials of the formulae as indicated in claim 1, or

c) condensing starting materials of the formulae as indicated in claim 1 and hydrolysing the resultant imines, or

d) isomerizing compounds of the formula as indicated in claim 1 with a strong base and, if desired, converting a resultant compound of formula I into another compound of the invention, and/or, if desired, converting a resultant enol into a $C_1$—$C_7$alkyl enol ether, and/or, if desired, converting a resultant enol into a salt with a base, or a resultant enol salt into the free enol or into another salt with a base, and/or, if desired, resolving a mixture of isomers obtained into the individual isomers.

10. A process according to claim 9, wherein the reactions a), b), c) and d) are carried out in accordance with any one of claims 2, 3, 4 and 5.

11. A process according to either claim 9 or claim 10, wherein in a resultant compound having a nitro group as substituent in the Ph radical, said group is reduced to the amino group.

12. A process according to either claim 9 or claim 10, which process comprises preparing a compound of the general formula II

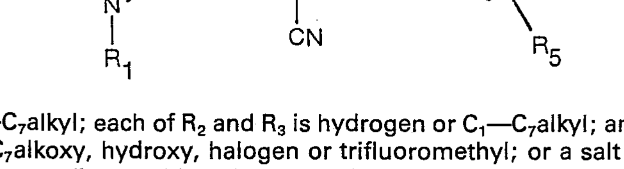 (II),

wherein $R_1$ is $C_1$—$C_7$alkyl; each of $R_2$ and $R_3$ is hydrogen or $C_1$—$C_7$alkyl; and each of $R_4$ and $R_5$ is hydrogen, $C_1$—$C_7$alkyl, $C_1$—$C_7$alkoxy, hydroxy, halogen or trifluoromethyl; or a salt thereof with a base.

13. A process according to either claim 9 or claim 10, which process comprises preparing a compound of formula II as indicated in claim 12, wherein $R_1$ is alkyl of not more than 4 carbon atoms; each of $R_2$ and $R_3$

is hydrogen; and each of $R_4$ and $R_5$ is hydrogen, alkyl or alkoxy of not more than 4 carbon atoms each, fluoro, chloro or trifluoromethyl, or a salt thereof with a base.

14. A process according to either claim 9 or claim 10, which process comprises preparing a compound of the formula II as indicated in claim 12, wherein $R_1$ is methyl; each of $R_2$ and $R_3$ is hydrogen; and each of $R_4$ and $R_5$ is hydrogen, methyl, methoxy, fluoro, chloro or trifluoromethyl, or a salt thereof with a base.

15. A process according to either claim 9 or claim 10, which process comprises preparing 1-methyl-β-oxo-α-phenylcarbamoyl-2-pyrrolepropionitrile, or a salt thereof with a base.

16. A process according to either claim 9 or claim 10, which process comprises preparing 1-methyl-β-oxo-α-(p-fluorophenylcarbamoyl)-2-pyrrolepropionitrile, or a salt thereof with a base.

17. A process for the preparation of a pharmaceutical composition, which process comprises combining a compound of the invention according to any one of claims 1 to 8 with a pharmaceutical carrier.

18. A process for the preparation of a pharmaceutical composition, which process comprises combining a compound of the invention according to any one of claims 9 to 16 with a pharmaceutical carrier.

**Revendications pour les Etats contractants: DE GB FR CH LI IT NL BE SE LU**

1. Bêta-oxo-alpha-phénylcarbamoyl-pyrrolopropionitriles substituées de formule générale I

$$\begin{array}{c} \qquad\qquad CN \\ \qquad\qquad | \\ Pl—CO—CH—CONH—Ph \end{array} \qquad (I),$$

dans laquelle Pl représente un reste 2- ou 3-pyrrolyle qui est substitué en position 1 par un groupe alkyle en $C_1$—$C_7$ ou phényl-alkyle en $C_1$—$C_7$ et est non substitué dans les trois autres positions ou est substitué par 1 à 3 groupes alkyle en $C_1$—$C_7$ identiques ou différents et/ou un groupe carboxy ou carbo-alcoxy en $C_1$—$C_7$; et Ph représente le groupe phényle non substitué ou portant un à trois substituants identiques ou différents choisis parmi les groupes alkyle en $C_1$—$C_7$, alkylthio en $C_1$—$C_7$, hydroxy, les halogènes, les groupes trifluorométhyle, nitro, amino ou alcanoylamino en $C_1$—$C_7$; ces composés sous leur forme énol, leurs éthers alkaliques en $C_1$—$C_7$-7 d'énols ou leurs esters alcanoyliques en $C_1$—$C_7$ d'énols ou leurs sels de bases.

2. Composés selon la rev. 1, de formule I, dans laquelle Pl représente un groupe 1-(alkyle en $C_1$—$C_7$ ou Ph-alkyle en $C_1$—$C_7$)-2- ou -3-pyrrolyle non substitué dans les autres positions ou substitué pa;r un ou deux groupes alkyle en $C_1$—$C_7$, un ou groupe carboxy ou carboalcoxy en $C_1$—$C_7$, Ph représente le groupe phényle non substitué ou portant un ou deux substituants identiques ou différents choisis parmi les groupes alkyle en $C_1$—$C_7$, alcoxy en $C_1$—$C_7$, alkylthio en $C_1$—$C_7$, hydroxy, les halogènes, les groupes trifluorométhyle, nitro, amino et alcanoylamino en $C_1$—$C_7$, leurs éthers alkyliques en $C_1$—$C_7$ d'énols ou leurs esters alcanoyliques en $C_1$—$C_7$ d'énols ou leurs sels de bases.

3. Composés selon la rev. 1, de formule I dans laquelle Pl représente un groupe 2- ou 3-pyrrolyle substitué en position 1 par un groupe alkyle en $C_1$—$C_7$ ou Ph-alkyle en $C_1$—$C_7$ et non substitué dans les trois autres positions ou portant un à trois substituants alkyle en $C_1$—$C_7$ identiques ou différents; et Ph représente le groupe phényle non substitué ou portant un à trois substituants identiques ou différents choisis parmi les groupes alkyle en $C_1$—$C_7$, alcoxy en $C_1$—$C_7$, alkylthio en $C_1$—$C_7$, hydroxy, les halogènes, les groupes trifluorométhyle, nitro ou amino, leurs éthers alkyliques en $C_1$—$C_7$ d'énols ou leurs sels de bases.

4. Composés selon la rev. 1 répondant à la formule générale II

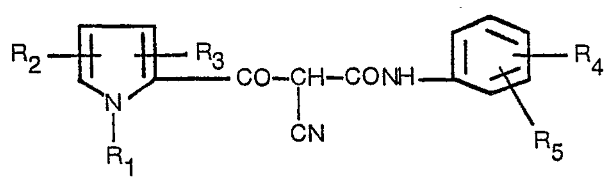

(II),

dans laquelle $R_1$ représente un groupe alkyle en $C_1$—$C_7$, chacun des symboles $R_2$ et $R_3$ représente l'hydrogène ou un groupe alkyle en $C_1$—$C_7$ et chacun des symboles $R_4$ et $R_5$ représente l'hydrogène, un groupe alkyle en $C_1$—$C_7$, alcoxy en $C_1$—$C_7$, hydroxy, un halogène ou un groupe trifluorométhyle, ou leurs sels de bases.

5. Composés selon la revendication 1, répondant à la formule II de la rev. 4, dans laquelle $R_1$ représente un groupe alkyle à 4 atomes de carbone au maximum, chacun des symboles $R_2$ et $R_3$ représente l'hydrogène et chacun des symboles $R_4$ et $R_5$ représente l'hydrogène, un groupe alkyle ou alcoxy contenant chacun 4 atomes de carbone au maximum, le fluor, le chlore ou un groupe trifluorométhyle, ou leurs sels de bases.

6. Composés selon la rev. 1, répondant à la formule II de la rev. 4 dans laquelle $R_1$ représente un groupe méthyle, chacun des symboles $R_2$ et $R_3$ représente l'hydrogène et chacun des symboles $R_4$ et $R_5$ représente

l'hydrogène, un groupe méthyle, méthoxy, le fluor, le chlore ou un groupe trifluorométhyle, ou leurs sels de bases.

7. Le 1-méthyl-bêta-oxo-alpha-phénylcarbamoyl-2-pyrrolopropionitrile et ses sels de bases.

8. Le 1-méthyl-bêta-oxo-alpha-(p-fluorophénylcarbamoyl)-2-pyrrolopropionitrile et ses sels de bases.

9. Composés à activité anti-inflammatoire et anti-arthritique selon la rev. 1.

10. Composés à activité anti-inflammatoire et anti-arthritique selon la rev. 3.

11. Compositions pharmaceutiques contenant des composés selon l'une des rev. 1, 2 et 9, les sels étant uniquement ceux qui conviennent pour l'usage pharmaceutique.

12. Compositions pharmaceutiques contenant des composés selon l'une des rev. 3 à 8 et 10, les sels étant uniquement ceux qui conviennent pour l'usage pharmaceutique.

13. Utilisation de composés selon les rev. 1, 2 et 9, pour la préparation de compositions pharmaceutiques.

14. Utilisation de composés selon les rev. 3 à 8 et 10, pour la préparation de compositions pharmaceutiques.

15. Les composés des rev. 1, 2 et 9, pour l'utilisation dans un procédé pour le traitement thérapeutique de l'organisme humain ou animal.

16. Les composés des rev. 3 à 8 et 10, pour l'utilisation dans un procédé pour le traitement thérapeutique de l'organisme humain ou animal.

17. Procédé de préparation des bêta-oxo-alpha-phénylcarbamoyl-pyrrolopropionitrile substitués définis dans la rev. 1, caractérisé en ce que:

a) on fixe par addition les composés de formule

$$Pl—CO—CH_2—CN \text{ et } OC=N—Ph$$

ou bien

b) on condense des composés de formules

Pl—CO—CH—COX et H—NH—Ph,
|
CN

dans lesquelles X représente un groupe alcoxy en $C_1—C_7$, alcanoyloxy en $C_1—C_7$ ou un halogène, ou bien

c) on condense des composés de formules

Pl—Y et $CH_2$—CONH—Ph
|
CN

dans lesquelles Y représente un groupe (alcoxy en $C_1—C_7$)-carbonyle, halogénocarbonyle ou cyano, et on hydrolyse les imines obtenues, ou bien

d) on isomérise des composés de formule

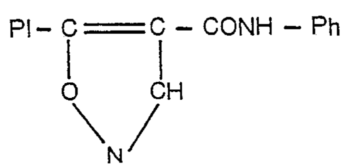

à l'aide d'une base forte, et si on le désire, on convertit un composé obtenu répondant à la formule I en un autre composé de l'invention et/ou, si on le désire, on convertit un énol obtenu en un éther alkylique en $C_1—C_7$ d'énol ou un ester alcanoylique en $C_1—C_7$ d'énol et/ou, si on le désire, on convertit un énol obtenu en un sel d'une base ou un sel d'énol obtenu en l'énol libre ou en un autre sel d'une base et/ou, si on le désire, on sépare un mélange d'isomères obtenu en les isomères individuels.

18. Les composés obtenus par le procdé de la rev. 17.

**Revendications pour l'Etat contractant: AT**

1. Procédé de préparation de bêta-oxo-alpha-phénylcarbamoyl-pyrrolopropionitriles de formule générale I

CN
|
Pl—CO—CH—CONH—Ph (I),

dans laquelle Pl représente un groupe 2- ou 3-pyrrolyle substitué en position 1 par un groupe alkyle en $C_1—C_7$ ou Ph-alkyle en $C_1—C_7$ et non substitué dans les trois autres positions ou substitué par un à trois

groupes alkyle en $C_1$—$C_7$ identiques ou différents et/ou un groupe carboxy ou carbo-alcoxy en $C_1$—$C_7$; et Ph représente le groupe phényle non substitué ou portant un à trois substituants identiques ou différents choisis parmi les groupes alkyle en $C_1$—$C_7$, alcoxy en $C_1$—$C_7$, alkylthio en $C_1$—$C_7$, hydroxy, les halogènes, les groupes trifluorométhyle, nitro, amino ou alcanoylamino en $C_1$—$C_7$, de ces composés sous leur forme d'énols, de leurs éthers alkyliques en $C_1$—$C_7$ d'énols ou de leurs esters alcanoyliques en $C_1$—$C_7$ d'énols ou de leurs sels de bases, caractérisé en ce que:

a) on fixe par addition les composés de formules

$$Pl—CO—CH_2—CN \text{ et } OC{=}N—Ph$$

b) on condense les composés de formules

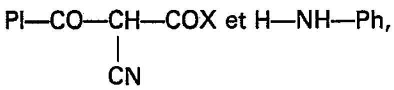

dans lesquelles X représente un groupe alcoxy en $C_1$—$C_7$, alcanoyloxy en $C_1$—$C_7$ ou un halogène, ou bien

c) on condense les composés de formules

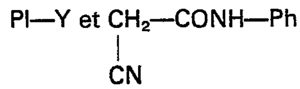

dans lesquelles Y représente un groupe (alcoxy en $C_1$—$C_7$)-carbonyle, halogénocarbonyle ou cyano, et on hydrolyse les imines obtenues, ou bien

d) on isomérise les composés de formule

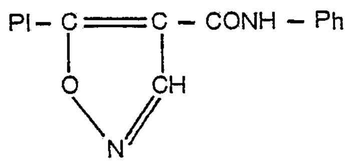

à l'aide d'une base forte, et si on le désire, on convertit un composé obtenu répondant à la formule I en un autre composé de l'invention et/ou, si on le désire, on convertit un énol obtenu en un éther alkylique en $C_1$—$C_7$ d'énol ou un ester alcanoylique en $C_1$—$C_7$ d'énol et/ou, si on le désire, on convertit un énol obtenu en un sel d'une base ou un sel d'énol obtenu en l'énol libre ou en un autre sel de base et/ou, si on le désire, on sépare un mélange d'isomères obtenu en les isomères individuels.

2. Procédé selon la rev. 1, caractérisé en ce que la réaction a) est effectuée en présence d'une base ou à température élevée.

3. Procédé selon la rev. 1, caractérisé en ce que la réaction b) est effectuée à température élevée.

4. Procédé selon la rev. 1, caractérisé en ce que la réaction c) est effectuée en présence d'un alcoolate ou d'un hydrure de métal alcalin.

5. Procédé selon la revendication 1, caractérisé en ce que la réaction d) est effectuée en présence d'une base minérale ou organique forte.

6. Procédé selon la rev. 1, caractérisé en ce que dans un composé obtenu dans lequel le groupe Ph porte en tant que substituant un groupe nitro, on réduit ce groupe en groupe amino.

7. Procédé selon l'une des rev. 1 à 6, caractérisé en ce que, dans un composé obtenu dans lequel le groupe Ph porte en tant que substituant le groupe amino, on acyle ce groupe en un groupe alcanoylamino en $C_1$—$C_7$.

8. Procédé selon l'une des rev. 1 à 7, caractérisé en ce que l'on prépare des composés de formule I de la rev. 1 dans laquelle Pl représente un groupe 1-(alkyle en $C_1$—$C_7$ ou Ph-alkyle en $C_1$—$C_7$)-2 ou -3-pyrrolyle non substitué dans les autres positions ou substitué par un ou deux groupes alkyle en $C_1$—$C_7$ ou par un groupe carboxy ou carbo-alcoxy en $C_1$—$C_7$, Ph représente le groupe phényle non substitué ou portant un ou deux substituants identiques ou différents choisis parmi les groupes alkyle en $C_1$—$C_7$, alcoxy en $C_1$—$C_7$, alkylthio en $C_1$—$C_7$, hydroxy, les halogènes, les groupes trifluorméthyl, nitro, amino et alcanoylamino en $C_1$—$C_7$, leurs éthers alkyliques en $C_1$—$C_7$ d'énols ou leurs esters alcanoyliques en $C_1$—$C_7$ d'énols ou leurs sels de bases.

9. Procédé selon la rev. 1, pour la préparation des nouveaux bêta-oxo-alpha-phénylcarbamoyl-pyrrolopropionitriles substitués de formule I de la rev. 1 dans laquelle Pl représente un groupe 2- ou 3-pyrrolyle substitué en position 1 par un groupe alkyle en $C_1$—$C_7$ ou Ph-alkyle en $C_1$—$C_7$ et non substitué dans les trois autres positions ou substitué par un à trois groupes alkyle en $C_1$—$C_7$ identiques ou différents; et Ph représente le groupe phényle non substitué ou portant un à trois substituants identiques ou différents choisis parmi les groupes alkyle en $C_1$—$C_7$, alcoxy en $C_1$—$C_7$, alkylthio en $C_1$—$C_7$, hydroxy, les halogènes,

les groupes trifluorométhyle, nitro ou amino; de leurs éthers alkyliques en $C_1$—$C_7$ d'énols ou de leurs sels de bases, caractérisés en ce que:

a) on fixe par addition des composés de départ répondant aux formules indiquées dans la rev. 1, ou bien

b) on condense des produits de départ répondant aux formules indiquées dans la rev. 1,

c) on condense des produits de départ répondant aux formules indiquées dans la rev. 1, et on hydrolyse les imines obtenues, ou bien

d) on isomérise des composés répondant à la formule indiquée dans la rev. 1 à l'aide d'une base forte, et, si on le désire, on convertit un composé obtenu répondant à la formule I en un autre composé de l'invention et/ou, si on le désire, on convertit un énol obtenu en un éther alkylique en $C_1$—$C_7$ d'énol, et/ou, si on le désire, on convertit un énol obtenu en un sel de base ou un sel d'énol obtenu en l'énol libre ou en un autre sel de base, et/ou, si on le désire, on sépare un mélange d'isomères obtenu en les isomères individuels.

10. Procédé selon la rev. 9, caractérisé en ce que l'on effectue les réactions a), b), c) et d) selon les rev. respectives 2, 3, 4 et 5.

11. Procédé selon l'une des rev. 9 et 10, caractérisé en ce que, dans un composé obtenu dans lequel le groupe Ph porte en tant que substituant un groupe nitro, on réduit ce groupe en un groupe amino.

12. Procédé selon l'une des rev. 9 et 10, caractérisé en ce que l'on prépare les composé de formule générale II

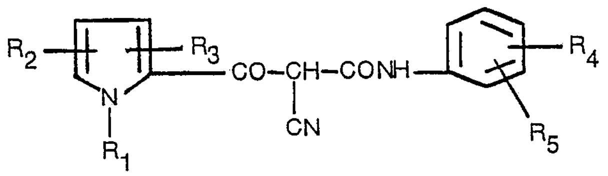
(II),

dans laquelle $R_1$ représente un groupe alkyle en $C_1$—$C_7$, chacun des symboles $R_2$ et $R_3$ représente l'hydrogène ou un groupe alkyle en $C_1$—$C_7$ et chacun des symboles $R_4$ et $R_5$ représente l'hydrogène, un groupe alkyle en $C_1$—$C_7$, alcoxy en $C_1$—$C_7$, hydroxy, un halogène ou un groupe trifluorométhyle, ou leurs sels de bases.

13. Procédé selon l'une des rev. 9 et 10, caractérisé en ce que l'on prépare des composés de formule II de la rev. 12, dans laquelle $R_1$ représente un groupe alkyle à 4 atomes de carbone au maximum, chacun des symboles $R_2$ et $R_3$ représente l'hydrogène et chacun des symboles $R_4$ et $R_5$ l'hydrogène, un groupe alkyle ou alcoxy ayant chacun 4 atomes de carbone au maximum, le fluor, le chlore ou un groupe trifluorométhyle, ou leurs sels de bases.

14. Procédé selon l'une des rev. 9 et 10, caractérisé en ce que l'on prépare des composés de formule II de la rev. 12, dans laquelle $R_1$ représente un groupe méthyle, chacun des symboles $R_2$ et $R_3$ l'hydrogène et chacun des symboles $R_4$ et $R_5$ l'hydrogène, un groupe méthyle, méthoxy, le fluor, le chlore ou un groupe trifluorométhyle, ou leurs sels de bases.

15. Procédé selon l'une des rev. 9 et 10, caractérisé en ce que l'on prépare le 1-méthyl-bêta-oxo-alpha-phénylcarbamoyl-2-pyrrolopropionitrile et ses sels de bases.

16. Procédé selon l'une des rev. 9 et 10, caractérisé en ce que l'on prépare le 1-méthyl-bêta-oxo-alpha-(p-fluorophénylcarbamoyl)-2-pyrrolopropionitrile et ses sels de bases.

17. Procédé de préparation d'une composition pharmaceutique, caractérisé en ce que l'on utilise à cet effet une substance active selon l'invention selon l'une des rev. 1 à 8, et un véhicule pharmaceutique.

18. Procédé de préparation d'une composition pharmaceutique caractérisé en ce que l'on utilise à cet effet une substance active selon l'invention selon l'une des rev. 9 à 16, et un véhicule pharmaceutique.